# EUROPEAN PATENT APPLICATION

(11) **EP 4 763 189 A1**
(43) Date of publication of application: **24.06.2026**
(21) Application number: 24221573.9
(22) Date of filing: 19.12.2024
(51) Int. Cl.: A61K 9/00, A61K 9/19, A61K 9/51, A61K 38/46, A61K 38/47, A61K 38/48, A61K 38/54

(54) **NANOPARTICLE COMPOSITIONS COMPRISING PANCREATIC ENZYMES**

(71) Applicant: Perseo Pharma AG, 4132 Muttenz (CH)
(72) Inventor: BRIAND, Manon, 5408 Ennetbaden (CH)
(74) Representative: Latscha Schöllhorn Partner AG

(57) **Abstract**

The present invention relates to a composition comprising a solid carrier, a lipase or a fragment thereof immobilized on the surface of the solid carrier wherein the lipase or a fragment thereof is in the open conformation, a protease or a fragment thereof immobilized on the surface of the solid carrier, an amylase or a fragment thereof immobilized on the surface of the solid carrier, an agent which interacts with the lid domain of the lipase or a fragment thereof, a protective layer to protect the lipase or a fragment thereof, the protease or a fragment thereof and the amylase or a fragment thereof by embedding the lipase or a fragment thereof, the protease or a fragment thereof and the amylase or a fragment thereof, and a functional constituent immobilized on the surface of the protective layer, wherein the functional constituent immobilized on the surface of the protective layer is a polymer comprising repeat units wherein each repeat unit comprises at least one amino group and/or at least one thiol group; b) an amino acid; c) a non-reducing sugar and/or a non-reducing sugar alcohol; and d) a surfactant. The present invention also relates to solid, lyophilized and liquid compositions of said nanoparticle composition, capsules comprising said nanoparticle composition, methods of producing said nanoparticle composition and uses thereof.

## Description

### The field of the invention

The present invention relates to a nanoparticle composition comprising a) a nanoparticle comprising a solid carrier, a lipase or a fragment thereof immobilized on the surface of the solid carrier wherein the lipase or a fragment thereof is in the open conformation, a protease or a fragment thereof immobilized on the surface of the solid carrier, an amylase or a fragment thereof immobilized on the surface of the solid carrier, an agent which interacts with the lid domain of the lipase or a fragment thereof, a protective layer to protect the lipase or a fragment thereof, the protease or a fragment thereof and the amylase or a fragment thereof by embedding the lipase or a fragment thereof, the protease or a fragment thereof and the amylase or a fragment thereof, and a functional constituent immobilized on the surface of the protective layer, wherein the functional constituent immobilized on the surface of the protective layer is a polymer comprising repeat units wherein each repeat unit comprises at least one amino group and/or at least one thiol group; b) an amino acid; c) a non-reducing sugar and/or a non-reducing sugar alcohol; and d) a surfactant. The present invention also relates to solid, lyophilized and liquid compositions of said nanoparticle composition, capsules comprising said nanoparticle composition, methods of producing said nanoparticle composition and uses thereof.

### Background of the invention

Exocrine Pancreatic Insufficiency (EPI) is a condition in which the exocrine functions of the pancreas are impaired and its ability to effectively deliver digestive enzymes to the duodenum is defective. It is a serious health condition that leads to malabsorption of lipids, fat-soluble vitamins, proteins, and, to a lesser extent, carbohydrates from the digestive tract.

The standard medical therapy to treat clinical symptoms and malabsorption is oral pancreatic enzyme replacement therapy (PERT). Current approved therapies consist in pancreatic enzyme product (lipase, amylase, protease) from porcine origin, also called pancreatin. A minimum dose of 40 000 - 50 000 Units of PERT-lipase is recommended at each main meals and half that dose with snacks. Such doses lead to a heavy pill burden for the patients. However, the current treatment is not efficient and exhibits several limitations: Inefficiency of the treatment, persistence of the symptoms, low intraluminal survival time of the lipase that is susceptible to proteases degradation (default of fat digestion), possible intolerance to massive loads of enzymes, and/or gastrointestinal troubles due to the high amount of proteases ingested. Thus, there is a need to provide compatible and effective compositions for treatment of Exocrine Pancreatic Insufficiency (EPI) in particular stable compositions to ensure optimal drug delivery to the duodenum.

### Summary of the invention

The present invention provides a nanoparticle composition comprising a) a nanoparticle comprising a solid carrier, a lipase or a fragment thereof immobilized on the surface of the solid carrier wherein the lipase or a fragment thereof is in the open conformation, a protease or a fragment thereof immobilized on the surface of the solid carrier, an amylase or a fragment thereof immobilized on the surface of the solid carrier, an agent which interacts with the lid domain of the lipase or a fragment thereof, a protective layer to protect the lipase or a fragment thereof, the protease or a fragment thereof and the amylase or a fragment thereof by embedding the lipase or a fragment thereof, the protease or a fragment thereof and the amylase or a fragment thereof, and a functional constituent immobilized on the surface of the protective layer, wherein the functional constituent immobilized on the surface of the protective layer is a polymer comprising repeat units wherein each repeat unit comprises at least one amino group and/or at least one thiol group; b) an amino acid; c) a non-reducing sugar and/or a non-reducing sugar alcohol; and d) a surfactant.

The present invention also provides a solid composition comprising the nanoparticle composition of the present invention.

The present invention also provides a lyophilized composition comprising the nanoparticle composition of the present invention.

The present invention also provides a liquid composition comprising the nanoparticle composition of the present invention.

The present invention also provides capsules comprising the nanoparticle composition of the present invention.

The present invention also provides a method of producing a nanoparticle composition, the composition comprising a) a nanoparticle comprising a solid carrier, a lipase or a fragment thereof immobilized on the surface of the solid carrier wherein the lipase or a fragment thereof is in the open conformation, a protease or a fragment thereof immobilized on the surface of the solid carrier, an amylase or a fragment thereof immobilized on the surface of the solid carrier, an agent which interacts with the lid domain of the lipase or a fragment thereof, a protective layer to protect the lipase or a fragment thereof, the protease or a fragment thereof, and the amylase or a fragment thereof by embedding the lipase or a fragment thereof, the protease or a fragment thereof and the amylase or a fragment thereof, and a functional constituent immobilized on the surface of the protective layer, wherein the functional constituent immobilized on the surface of the protective layer is a polymer comprising repeat units wherein each repeat unit comprises at least one amino group and/or at least one thiol group; b) an amino acid; c) a non-reducing sugar and/or a non-reducing sugar alcohol; and d) a surfactant; the method comprising the following steps:
(a) providing a solid carrier;
(b) providing a lipase or a fragment thereof, a protease or a fragment thereof and an amylase or a fragment thereof;
(c) providing an agent which interacts with the lid domain of a lipase or a fragment thereof;
(d) allowing the lipase or a fragment thereof of (b) to interact with the agent of (c);
(e) immobilizing the lipase or a fragment thereof, the protease or a fragment thereof and the amylase or a fragment thereof on the solid carrier;
(f) forming a protective layer on the surface of the solid carrier to protect the lipase or a fragment thereof, the protease or a fragment thereof and the amylase or a fragment thereof immobilized on the solid carrier;
(g) immobilizing a functional constituent on the surface of the protective layer to obtain the nanoparticle, wherein the functional constituent immobilized on the surface of the protective layer is a polymer comprising repeat units, wherein each repeat unit comprises at least one amino group and/or at least one thiol group;
(h) providing the nanoparticles obtained in (g) in suspension and adding the amino acid of b); the non-reducing sugar and/or the non-reducing sugar alcohol of c) and the surfactant of d); and optionally;
(j) lyophilizing the supspension to obtain dry pellets and further optionally blending the dry pellets to obtain a dry powder; and optionally;
(k) loading the dry powder into capsules.

The present invention also provides a method for the prevention, delay of progression or treatment of exocrine pancreatic insufficiency (EPI) and a method of enzyme replacement therapy (ERT) using the nanoparticle composition of the present invention.

It has been surprisingly found by the inventors of the present application that the nanoparticle compositions as provided by the present invention provide for excellent preservation of enzymatic activity post-freeze-drying, with 100% retention of lipase activity, 58% retention of amylase activity, and 63% retention of protease activity. Even more surprisingly the freeze-drying process does not adversely affect the nature or shape of the nanoparticles and does not adversely affect the interaction between the nanoparticles and the mucus layer.

### Brief description of the figures

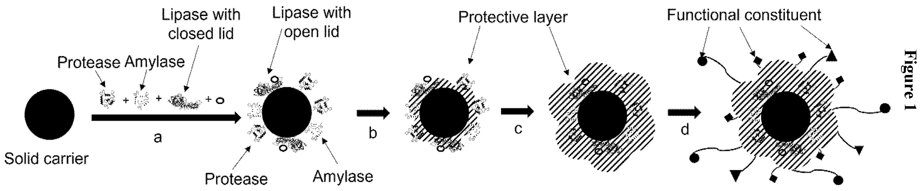

**Figure** 1) shows a schematic representation of the process for the production of the composition of the invention: a) to a solid carrier, a lipase with closed lid, a protease, an amylase and an agent (displayed as round circle) which interacts with the lid domain of the lipase is provided and the lipase with an open lid, the protease and the amylase are immobilized on the solid carrier; b) and c) a protective layer grows around the immobilized lipase with an open lid, the protease and the amylase, embedding all three enzymes) and d) a functional constituent is immobilized on the surface of the protective layer.
**Figure** 2) Activity recovery of a) lipase, b) amylase and c) protease after freeze-drying of NP-3-DS.
**Figure** 3) Lyostat analysis to evaluate the impact of the freeze-drying process on the quality of non-formulated NP-3 suspension showing a) a good structure of NP-3 suspension at -80°C, b) first sign of NP-3 suspension collapse at -55.3°C, c) total NP-3 suspension collapse at -45.3°C.
**Figure** 4) Lyostat analysis to evaluate the impact of the freeze-drying process on the quality of NP-3-DS showing a) a good structure of NP-3-DS at -80°C, b) first sign of NP-3-DS collapse at -33°C, c) total NP-3-DS collapse at -28.2°C.
**Figure** 5) Residual moisture content of dry NP-3 measured by Karl Fischer titration.
**Figure** 6) Scanning electron micrographs of a) NP-3-DS and b) dry NP-3.
**Figure** 7) Dry NP-3 characterization. a) Macroscopic appearance of dry NP-3, b) Dry NP-3 appearance by binocular lens, c) Particle size distribution of dry NP-3.
**Figure** 8) illustrates the ex-vivo interaction of fluorescent NP-3 nanoparticles with a porcine mucus layer. Nanoparticles (500 µg/mL) were applied as either powder (NP-3-DP) or suspension (NP-3-DS) and incubated for 1 hour at37°C. Following incubation, the mucus layer was sequentially washed with water, NaCl solution, and Triton X-100. The histograms depict the percentage of nanoparticles remaining bound to the mucus after each washing step.
**Figure** 9) shows the kinetics of eudracap capsule dissolution in a) FEDGAS pH 6, b) FEDGAS pH 4.5, c) FEDGAS pH 3 and d) FeSSIF-V2.
**Figure** 10) Dog weight variation following (A) PDL surgery, (B) Untreated Period, (C) Single-Dose Treatment, and (D) Chronic Treatment for 3.5 days.
Figure 11) Fecal fat analysis. a) Fecal fat content (%) and b) Fatty digestion efficacy (%) of untreated dogs, dogs treated with NP-3-DP for 3 days, dogs treated with Panzym for 3 days and healthy controls.

### Detailed description of the invention

The present invention relates to a nanoparticle composition comprising a) a nanoparticle comprising a solid carrier, a lipase or a fragment thereof immobilized on the surface of the solid carrier wherein the lipase or a fragment thereof is in the open conformation, a protease or a fragment thereof immobilized on the surface of the solid carrier, an amylase or a fragment thereof immobilized on the surface of the solid carrier, an agent which interacts with the lid domain of the lipase or a fragment thereof, a protective layer to protect the lipase or a fragment thereof, the protease or a fragment thereof and the amylase or a fragment thereof by embedding the lipase or a fragment thereof, the protease or a fragment thereof and the amylase or a fragment thereof, and a functional constituent immobilized on the surface of the protective layer, wherein the functional constituent immobilized on the surface of the protective layer is a polymer comprising repeat units wherein each repeat unit comprises at least one amino group and/or at least one thiol group; b) an amino acid; c) a non-reducing sugar and/or a non-reducing sugar alcohol; and d) a surfactant.

For the purposes of interpreting this specification, the following definitions will apply and whenever appropriate, terms used in the singular will also include the plural and *vice versa.* It is to be understood that the terminology used herein is for the purpose of describing particular embodiments only and is not intended to be limiting.

Features, integers, characteristics, compounds described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process disclosed, may be combined in any combination, except combinations where at least some of such features and/or steps are mutually exclusive. The invention is not restricted to the details of any foregoing embodiments.

The term "comprise" and variations thereof, such as, "comprises" and "comprising" is generally used in the sense of include, that is, as "including, but not limited to", that is to say permitting the presence of one or more features or components.

The term "contain" and variations thereof, such as, "contains" and "containing" as used herein is generally used in the sense of "composed of substantially only," or "composed of only", that is to say not permitting the presence of other features or components than explicitly mentioned.

The singular forms "a," "an," and "the" include plural referents unless the content clearly dictates otherwise.

The term "about" refers to a range of values ± 10% of a specified value. For example, the phrase "about 200" includes ± 10% of 200, or from 180 to 220.

The term "nanoparticle" as used herein refers usually to a particle which is usually between 10 nm and 1000 nm, preferably between 15 nm and 500 nm, more preferably between 20 nm and 200 nm, in particularly between 40 nm and 100 nm.

The term "solid carrier" as used herein refers usually to a particle. Preferably the solid carrier is a monodisperse particle or a polydisperse particle, more preferably a monodisperse particle. The solid carrier usually comprises organic particles, inorganic particles, organic-inorganic particles, self-assembling organic particles, silica particles, gold particles, titanium particles and is preferably a silica particle, more preferably a silica nanoparticle (SNP). The particle size of the solid carrier is usually between 1 nm and 1000 nm, preferably between 10 nm and 100 nm, particularly about 50 nm.

The term "linker" or "cross-linker" which are used synonymously herein refers to any linking reagents containing groups, which are capable of binding to specific functional groups (e.g. primary amines, sulfhydryls, etc.). A linker in the context of the present invention usually connects the surface of the solid carrier with the enzyme e.g. the lipase or a fragment thereof, the protease or a fragment thereof and the amylase or a fragment thereof. For example, a linker may be immobilized on the surface of the solid carrier e.g. on the silica surface as a carrier material and then the enzyme e.g. the lipase or a fragment thereof, the protease or a fragment thereof and the amylase or a fragment thereof may be bound to an unoccupied binding-site of the linker. Alternatively, the linker may firstly bind to the enzyme e.g. the lipase or a fragment thereof, the protease or a fragment thereof and the amylase or a fragment thereof and then the linker bound to the enzyme e.g. the lipase or a fragment thereof, the protease or a fragment thereof and the amylase or a fragment thereof may bind with its unoccupied binding-site to the solid carrier. Various types of linkers are known in the art, including but not limited to straight or branched-chain carbon linkers, heterocyclic carbon linkers, peptide linkers, polyether linkers, and linkers that are known in the art as tags.

The term "protective layer" as used herein refers to a layer for protecting the functional properties of the protein or fragment thereof e.g. the lipase or a fragment thereof, the protease or a fragment thereof and the amylase or a fragment thereof immobilized on the surface of the solid carrier. The protective layer of the present invention is usually built with building blocks at least part of which are monomers capable of interacting with both each other usually by covalent binding and the immobilized protein or fragment thereof e.g. the lipase or a fragment thereof, the protease or a fragment thereof and the amylase or a fragment thereof usually by non-covalent binding. The protective layer is formed on the surface of the solid carrier to protect the protein or the fragment thereof e.g. the lipase or a fragment thereof, the protease or a fragment thereof and the amylase or a fragment thereof immobilized on the solid carrier. The protective layers are usually homogeneous layers where at least 50%, preferably at least 70%, more preferably at least 90% of the protein or fragment therof e.g. the lipase or a fragment thereof, the protease or a fragment thereof and the amylase or a fragment thereof are embedded in the protective layer.

The term, "lipase or a fragment thereof" includes naturally occurring lipases or a fragment thereof and also includes artificially engineered lipases or a fragment thereof. Artificially engineered lipases or a fragment thereof are e.g. variants or functionally active fragments of the lipase. The terms "fragment of a lipase", "fragment thereof' in relation to the lipase and "functionally active fragment of a lipase" are thus used synonymously herein. By "variants or functionally active fragments thereof' in relation to the lipase of the present invention is meant that the fragment or variant (such as an analogue, derivative or mutant) is capable of exercising the same physiological function as the lipase. Such variants include naturally occurring allelic variants and non-naturally occurring variants. Additions, deletions, substitutions and derivatizations of one or more of the amino acids are contemplated so long as the modifications do not result in loss of functional activity of the fragment or variant. Preferably the functionally active fragment or variant has at least about 80% sequence identity more preferably at least about 90% sequence identity, even more preferably at least about 95% sequence identity, most preferably at least about 98% sequence identity to the relevant part of the lipase. A fragment of a lipase as defined herein does usually have the same functional properties as the lipase i.e. the full length enzyme from which it is derived and includes at least the lid domain and the substrate binding region. A fragment of a lipase contains usually between 100 and 450 amino acids, preferably between 150 and 400 amino acids, more preferably between 200 and 350 amino acids. A preferred lipase or a fragment thereof is a lipase or a fragment thereof extracted from pancreas, more preferably extracted from pancreas of porcine origin, even more preferably a lipase or a fragment thereof comprised by pancreatin.

The term, "protease or a fragment thereof" includes naturally occurring proteases or a fragment thereof and also includes artificially engineered proteases or a fragment thereof. Artificially engineered proteases or a fragment thereof are e.g. variants or functionally active fragments of the protease. The terms "fragment of a protease", "fragment thereof' in relation to the protease and "functionally active fragment of a protease" are thus used synonymously herein. By "variants or functionally active fragments thereof" in relation to the protease of the present invention is meant that the fragment or variant (such as an analogue, derivative or mutant) is capable of exercising the same physiological function as the protease. Such variants include naturally occurring allelic variants and non-naturally occurring variants. Additions, deletions, substitutions and derivatizations of one or more of the amino acids are contemplated so long as the modifications do not result in loss of functional activity of the fragment or variant. Preferably the functionally active fragment or variant has at least about 80% sequence identity more preferably at least about 90% sequence identity, even more preferably at least about 95% sequence identity, most preferably at least about 98% sequence identity to the relevant part of the protease. A fragment of a protease as defined herein does usually have the same functional properties as the protease from which it is derived. A fragment of a protease contains usually between 50 and 200 amino acids, preferably between 75 and 175 amino acids, more preferably between 100 and 150 amino acids. A preferred protease or a fragment thereof is a protease or a fragment thereof extracted from pancreas, more preferably extracted from pancreas of porcine origin, even more preferably a protease or a fragment thereof comprised by pancreatin.

The term, "amylase or a fragment thereof" includes naturally occurring amylases or a fragment thereof and also includes artificially engineered amylases or a fragment thereof. Artificially engineered amylases or a fragment thereof are e.g. variants or functionally active fragments of the amylase. The terms "fragment of an amylase", "fragment thereof' in relation to the amylase and "functionally active fragment of an amylase" are thus used synonymously herein. By "variants or functionally active fragments thereof" in relation to the amylase of the present invention is meant that the fragment or variant (such as an analogue, derivative or mutant) is capable of exercising the same physiological function as the amylase. Such variants include naturally occurring allelic variants and non-naturally occurring variants. Additions, deletions, substitutions and derivatizations of one or more of the amino acids are contemplated so long as the modifications do not result in loss of functional activity of the fragment or variant. Preferably the functionally active fragment or variant has at least about 80% sequence identity more preferably at least about 90% sequence identity, even more preferably at least about 95% sequence identity, most preferably at least about 98% sequence identity to the relevant part of the amylase. A fragment of an amylase as defined herein does usually have the same functional properties as the amylase from which it is derived. A fragment of a amylase contains usually between 100 and 550 amino acids, preferably between 200 and 500 amino acids, more preferably between 300 and 450 amino acids. A preferred amylase or a fragment thereof is an amylase or a fragment thereof extracted from pancreas, more preferably extracted from pancreas of porcine origin, even more preferably an amylase or a fragment thereof comprised by pancreatin.

The term "pancreatin" also known as and used interchangeably herein with "pancreatic enzymes" as used herein refers to pancreatic enzyme preparation derived from porcine pancreatic glands and comprises a lipase or a fragment thereof, a protease or a fragment thereof and an amylase or a fragment thereof. The term "pancreatin" as used herein also comprises formulated pancreatic enzymes like capsules comprising pancreatic enzymes e.g. Zenpep ^{®}.

The term "partially embedded protein" as used herein shall mean that the protein e.g. the lipase or a fragment thereof, the protease or a fragment thereof and the amylase or a fragment thereof is not fully covered by the protective layer, thus, the protein e.g. the lipase or a fragment thereof, the protease or a fragment thereof and the amylase or a fragment thereof is not fully embedded in the protective layer. In one embodiment less than 50% of the protein e.g. the lipase or a fragment thereof, the protease or a fragment thereof and the amylase or a fragment thereof are covered by the protective layer, though typically more at least 70% will be covered, thus improving protection of the protein. In a preferred embodiment, at least 70%, more preferably at least 80%, even more preferably at least 90%, most preferably at least 95% of the protein e.g. the lipase or a fragment thereof, the protease or a fragment thereof and the amylase or a fragment thereof is covered by the protective layer. In another preferred embodiment, around 70% to around 95%, more preferrably around 80% to around 95%, even more preferably around 90% to around 95%, most preferably around 90% to around 95, 96, 97, 98 or 99 % of the protein e.g. the lipase or a fragment thereof, the protease or a fragment thereof and the amylase or a fragment thereof are covered by the protective layer. In a particularly preferred embodiment, around 70%, particularly around 80%, more particularly around 90%, most particularly around 95% of the protein e.g. the lipase or a fragment thereof, the protease or a fragment thereof and the amylase or a fragment thereof is covered by the protective layer. In a more particularly preferred embodiment, around 70%, particularly around 80%, more particularly around 90%, most particularly around 95% of the protein e.g. the lipase or a fragment thereof, the protease or a fragment thereof and the amylase or a fragment thereof is covered by the protective layer, wherein the active site is not covered.

The term "fully embedded protein" as used herein shall mean that the protein e.g. the lipase or a fragment thereof, the protease or a fragment thereof and the amylase or a fragment thereof according to the invention is fully, i.e. 100% covered by the protective layer, i.e. that also the active site is covered. Preferably the lipase or a fragment thereof, the protease or a fragment thereof and the amylase or a fragment thereof according to the invention are fully, i.e. 100% covered by the protective layer, i.e. that also the active site is covered.

The term "at least partially embedded protein" as used herein shall mean that the protein e.g. the lipase or a fragment thereof, the protease or a fragment thereof and the amylase or a fragment thereof is at least partially embedded and may be fully embedded by the protective layer. Thus "at least partially embedded protein" means that the protective layer covers from about 30% and 100% of the protein or a fragment therof e.g. the lipase or a fragment thereof, the protease or a fragment thereof and the amylase or a fragment thereof, preferably from about 50% to about 100%, more preferably from about 80% to about 100%, even more preferably from about 90% to about 100%, most preferably from about 95% to about 100 %, wherein the active site is preferably covered.

The term "agent which interacts with the lid domain of the lipase or a fragment thereof' as used herein refers to an agent which normally binds to the lid domain of the lipase or a fragment thereof and/or to the region of the lipase or a fragment thereof surrounding the lid domain, thereby causing the lid domain to shift the lipase or a fragment thereof to the open conformation and/or to maintain the open conformation of the lipase or the fragment thereof. The lid domain of lipases is normally an amphipathic structure; in the closed conformation, their hydrophilic side faces the solvent, while the hydrophobic side is directed toward the catalytic pocket (Brocca S., Secundo F., Ossola M., Alberghina L., Carrea G., Lotti M. (2003). Sequence of the lid affects activity and specificity of Candida rugosa lipase isoenzymes. Protein Sci. 12, 2312-2319. 10.1110/ps.0304003). As the lipase shifts to the open conformation, the hydrophobic face becomes exposed and contributes to the substrate-binding region. Preferably the agent which interacts with the lid domain of the lipase or a fragment thereof, causes the lipase or a fragment to be locked in its active conformation. When locked in its active conformation the lipase is normally fully activated. The agent which interacts with the lid domain of the lipase or a fragment thereof so that the the lipase or a fragment thereof is in the open conformation include a colipase or a fragment thereof, a colipase-mimicking peptide, and an amphipathic molecule.

The term "amphipathic molecule" as used herein refers to a molecule like a chemical compound containing both polar (water-soluble) and nonpolar (not water-soluble) portions in its structure. It may also relate to a molecule like a chemical compound having both hydrophobic and hydrophilic regions. Amphipathic molecules include bile salts, phospholipids, and nonionic detergents.

The terms" open conformation" or "open conformation of a lipase or a fragment thereof' which are used interchangeably herein refer to the conformation of the lipase or the fragment thereof where substrates can enter the lipases' active sites and be converted. In the closed conformation entrance of substrates to the active site of the lipase or a fragment thereof and its conversion is limited or not possible. The conformation of the lipase or fragment thereof i.e. whether the lipase is in open or closed confirmation can be determined by X-ray crystallography, enzymatic activity study, site-directed spin labeling (SDSL) methods and electron paramagnetic resonance (EPR).

The term "colipase or a fragment thereof" as used herein includes naturally occurring colipases or a fragment thereof and also includes artificially engineered colipases or a fragment thereof. Artificially engineered colipases or a fragment thereof are e.g. variants or functionally active fragments of the lipase. By "variants or functionally active fragments thereof" in relation to the colipase of the present invention is meant that the fragment or variant (such as an analogue, derivative or mutant) is capable of exercising the same physiological function as the colipase. Such variants include naturally occurring allelic variants and non-naturally occurring variants. Additions, deletions, substitutions and derivatizations of one or more of the amino acids are contemplated so long as the modifications do not result in loss of functional activity of the fragment or variant. Preferably the functionally active fragment or variant has at least about 80% sequence identity more preferably at least about 90% sequence identity, even more preferably at least about 95% sequence identity, most preferably at least about 98% sequence identity to the relevant part of the lipase. A fragment of a colipase as defined herein does have the same functional properties as the colipase from which it is derived. A preferred colipase is the colipase with Uniprot number: P02703.

The term "colipase mimicking peptide" as used herein refers to a peptide consisting of between 10 and 40 amino acids, allowing specific amino acid residues to be geometrically located in the right position to interact with amino acids of the pancreatic lipase structure, inducing stretching of the lipase conformation and opening of the lid, and thereby having the same functional properties as the colipase. A colipase mimicking peptide which can be used in the present invention is preferably the peptide as shown in SEQ ID NO: 1.

The term "bile salt" as used herein refers to bile acids conjugated with taurine or glycine and include sodium taurocholate, sodium glycocholate, sodium glycodeoxycholate, sodium taurodeoxycholate, sodium glycochenodeoxycholate, and sodium taurochenodeoxycholate.

The term "nonionic detergent" as used herein refers to a surfactant and include tetra ethylene glycol monooctyl ether, octyl-b-D-glucopyranoside, N,N-dimethyldodecylamine-N-oxide, and b-octylglucomaltoside.

The term "phospholipids" as used herein refers to a class of lipids whose molecule has a hydrophilic "head" containing a phosphate group and two hydrophobic "tails" derived from fatty acids, joined by an alcohol residue (usually a glycerol molecule). Phospholipids include lecithin and lysolecithin.

The term "functional constitutent" as used herein refers to a constituent which after being immobilized to the surface of the protective layer retains its characteristic, functional property. A functional constituent in the sense of the present invention is a polymer comprising repeat units wherein each repeat unit comprises at least one amino group and/or at least one thiol group.

The term "polymer comprising repeat units wherein each repeat unit comprises at least one amino group" as used herein refers to a polymer comprising a number of repeat units (monomers), whererin each repeat unit comprises at least one amino group. A preferred polymer comprises a number of repeat units (monomers), whererin each repeat unit contains one amino group, in particular one primary amino group.

The term "polymer comprising repeat units wherein each repeat unit comprises at least one thiol group" as used herein refers to a polymer comprising a number of repeat units (monomers), whererin each repeat unit comprises at least one thiol group. A preferred polymer comprises a number of repeat units (monomers), whererin each repeat unit contains one thiol group.

The term "polycarbophil-cysteine conjugates" as used herein refers to conjugates which comprise cysteine covalently attached to polycarbophil. Such conjugates can be produced as referred in e.g. Bernkop-Schnurch and Thaler, 2000, Journal of Pharmaceutical Sciences 89(7):901-9.

The term "polylysine" as used herein refers to α-polylysine and or ε-polylysine (ε-poly-L-lysine, EPL), preferably ε-polylysine. α-polylysine is a synthetic polymer, which can be composed of either L-lysine or D-lysine. ε-polylysine (ε-poly-L-lysine, EPL) is typically produced as a homopolypeptide of approximately 25-30 L-lysine residues.

The term "polycysteine" as used herein can be composed of either L-cysteine or D-cysteine and is preferably composed of L-cysteine and comprises preferably between 2 and 30 cysteine residues, more preferably between 2 and 5 cysteine residues.

The term "polyglucosamin" as used herein refers to linear amino-polysaccharides composed of D-glucosamine and N-acetyl-D-glucosamine units linked by (1-4) glycosidic bonds. Polyglucosamine contains free amine (-NH2) groups and may be characterized by the proportion of N-acetyl-D-glucosamine units and D-glucosamine units, which is expressed as the degree of deacetylation (DDA) of the fully acetylated polymer chitin. A preferred polyglucosamin of the present invention is selected from the group consisting of chitin, chitosan, polyglucosaminoglycans, chondroitin, heparin, keratan and dermatan or a derivative thereof. Most preferred is a chitosan or a derivative thereof.

The term "chitosan or a derivative thereof' as used herein refers to a chitosan or chitosan derivative thereof including a salt thereof which has preferably a molecular weight of 2 000 Da or more, preferably in the range 25 000 - 2 000 000 Da and more preferably about 50 000 - 350 000 Da, most preferably about 50 000 - 190 000 Da or 190 000 - 310 000 Da. The term chitosan derivatives includes ester, ether or other derivatives formed by reaction of acyl or alkyl groups with the OH groups. Examples are O-alkyl ethers of chitosan, O-acyl esters of chitosan. Suitable derivatives are given e.g. in G.A.E. Roberts, Chitin Chemistry, MacMillan Press Ltd, London, 1992. Suitable salts of chitosan include nitrates, phosphates, sulphates, xanthates, hydrochlorides, glutamates, lactates, acetates.

The term "amino acid" as used herein is used in its broadest sense and may refer to an amino acid in its many different chemical forms, its tautomeric, polymeric and/or isomeric forms, its analog forms, and/or its derivative forms. Amino acids comprise, by way of non-limiting example: Agmatine, Beta Alanine, Arginine, Asparagine, Aspartic Acid, Cysteine, Glutamine, Glutamic Acid, Glycine, Histidine, L-Histidine, Leucine, Isoleucine, Lysine, Methionine, Phenyl Beta Alanine, Proline, Serine, Threonine, Tryptophan, Tyrosine, Valine, Citrulline, Creatine, Glutamine, Norvaline, Omithine, and Phenylalanine. Usually the term" amino acid" as used herein comprise the 20 standard amino acids selected from the group consisting of Isoleucine(I) ,Valine(V), Leucine(L) , Phenylalanine(F) , Cysteine(C), Methionine(M) , Alanine(A), Glycine(G), Threonine(T), Serine(S), Tryptophan(W), Tyrosine(Y), Proline(P), Histidine(H), Asparagine(N), Asparatic acid(D), Glutamine(Q), Glutamic acid(E), Lysine(K) , and Arginine(R), preferably selected from the group consisting of Alanine, Arginine, Cysteine, Glutamic acid, Glutamine, Glycine, Histidine, Isoleucine, Leucine, Lysine, Methionine, Phenylalanine, Serine and Tryptophan.

The term "a non-reducing sugar and/or a non-reducing sugar alcohol" as used herein refers to a sugar and/or sugar alcohol that cannot act as a reducing agent due to the absence of a free aldehyde or ketone group. Examples of a non-reducing sugar and a non-reducing sugar alcohol include Glucose, Galactose, Lactose, Sucrose, Melbiose, Trehalose, Mannitol, Sorbitol, Erythritol, Glucosamine, Myo-inositol, Xylitol, Cyclodextrins, Stachyose, Lactosucrose, Melezidose, Raffinose, Alginate, Carrageenan, Chitosan, Chondroitin sulfate, Dextran, Inulin, Ficoll, Hyaluronan, and Carboxymethylcellulose. Preferably, a non-reducing sugar is comprised by the nanoparticle composition of the present invention.

The term "surfactant" as used herein refers to a compound that contains a lipophilic segment and a hydrophilic segment, which when added to water or solvents, reduces the surface tension of the system. Surfactants comprise nonionic surfactants and ionic surfactants. Non-ionic surfactants are usually selected from the group consisting of ethoxylated sorbitan esters like PEG-40 sorbitan diisostearate, polysorbate 80 (PS80), polysorbate 20 (PS20), polysorbate 40 (PS40), polysorbate 60 (PS60); block co-polymers like poloxamer 124, poloxamer 188, poloxamer 331, poloxamer 407, fatty acids ethoxylates like PEG-5 oleate, PEG-8 stearate, polyoxyl 40 stearate, polyoxyl 15 hydroxystearate, Brij 97, fatty alcohol ethoxylates like steareth 40; fatty acid esters like ascorbyl palmitate, beeswax, polyglyceryl 3-oleate, propylene glycol monocaprylate, propylene glycol monolaurate; fatty alcohols like cetostearyl alcohol, cetyl alcohol, myristic alcohol, stearyl alcohol; glycerides; pegylated triglycerides; and sugar esters. Ionic surfactants are are usually selected from the group consisting of sodium stearate, sodium cholate, sodium lauryl sulphate, cetylpyridinium chloride, and octenidine dihydrochloride. Examples of surfactants include Polysorbate 80, Polysorbate 20, Brij 97, Kolliphor EL, Poloxamer 407, Poloxamer 188, Sodium dodecyl sulfate, Sodium taurocholate, Tyloxapol, Polyvinylpyrrolidone 10K, Polyvinylpyrrolidone 40K.

The term "solid composition" as used herein refers to solids in crystalline, powder, and/or amorphous form, or any mixtures thereof. It is in the solid state at normal temperature and pressure (NTP) as defined by the US National Institute of Standards and Technology (NIST). The solid compositions described herein may contain (in addition to any water of hydration) trace amounts of water or other solvents, preferably less than 4 wt % of the water or other solvents.

The terms "lyophilized", "lyophilization", and "lyophilize" as used herein refer to a process of freeze-drying, where a sample is first frozen and dried, typically under a vacuum, to remove water and/or other solvents. The term "lyophilized composition" as used herein refers to a composition which has been freeze-dried.

The term "liquid composition" as used herein refers to a composition in liquid state and usually comprises the nanoparticles of the present invention in suspension in a buffer.

In a first aspect the present invention provides a nanoparticle composition comprising a) a nanoparticle comprising a solid carrier, a lipase or a fragment thereof immobilized on the surface of the solid carrier wherein the lipase or a fragment thereof is in the open conformation, a protease or a fragment thereof immobilized on the surface of the solid carrier, an amylase or a fragment thereof immobilized on the surface of the solid carrier, an agent which interacts with the lid domain of the lipase or a fragment thereof, a protective layer to protect the lipase or a fragment thereof, the protease or a fragment thereof and the amylase or a fragment thereof by embedding the lipase or a fragment thereof, the protease or a fragment thereof and the amylase or a fragment thereof , and a functional constituent immobilized on the surface of the protective layer, wherein the functional constituent immobilized on the surface of the protective layer is a polymer comprising repeat units wherein each repeat unit comprises at least one amino group and/or at least one thiol group; b) an amino acid; c) a non-reducing sugar and/or a non-reducing sugar alcohol; and d) a surfactant.

The protein or fragment thereof, e.g. the lipase or a fragment thereof, the protease or a fragment thereof and the amylase or a fragment thereof can be immobilized on the surface of the solid carrier by non-covalent binding or covalent binding. Non-covalent binding includes p-p (aromatic) interactions, van der Waals interactions, H-bonding interactions, and electrostatic interactions like e.g. ionic interactions. Preferably, the protein or fragment thereof, e.g. the lipase or a fragment thereof, the protease or a fragment thereof and the amylase or a fragment thereof is immobilized on the surface of the solid carrier by covalent binding or by covalent binding via a linker.

A solution of pancreatin, a lipase or a fragment thereof, a protease or a fragment and/or an amylase or a fragment thereof usually comprises the protein or a fragment thereof or the proteins or a fragment thereof in a buffer solution. Buffers which can be used are usually phosphate, chloride, citrate, MES, MOPS, HEPES, PIPES, ACES or mixtures thereof. The solution may additionally contain sugar alcohols or non-ionic surfactants as described herein. A solution of pancreatin, a lipase or a fragment thereof, a protease or a fragment and/or an amylase or a fragment thereof can be prepared by e.g. dissolving the protein or fragment or the proteins or a fragment thereof thereof in water to reconstitute the stock buffer of the protein or a fragment thereof or the proteins or a fragment thereof.

In one embodiment the solid carrier is selected from the group of organic particles, inorganic particles, organic-inorganic particles, self-assembling organic particles, silica particles, gold particles, titanium particles and is preferably a silica particle, more preferably a silica nanoparticle (SNP). The nanoparticle size is usually measured by measuring the diameter of the particles and is usually between 10 nm and 1000 nm, preferably between 15 nm and 500 nm, more preferably between 20 nm and 200 nm, in particularly between 50 nm and 100 nm. In case the solid carrier is a monodisperse particle, the size of the nanoparticle is usually between 10 nm and 1000 nm, preferably between 15 nm and 500 nm, more preferably between 20 nm and 200 nm, in particularly between 50 nm and 100 nm. In one embodiment the composition comprises a solid carrier wherein the solid carrier comprises at least 15%, preferably at least 25%, in particular between 15% and 40%, more particular between 25% and 35 % immobilized lipase, protease and/or amylase, or a fragment thereof, per dry weight of the solid carrier.

Usually monodisperse particles or polydisperse particles, preferably monodisperse particles are used as solid carrier in the present invention. In a preferred embodiment the monodisperse particles are spherical monodisperse particles.

The solid carrier is usually provided in suspension. Suspension of the solid carrier can be e.g. in water, buffer or non-ionic surfactants or mixtures thereof, preferably in mixtures of water and non-ionic surfactants. Non-ionic surfactants are usually selected from the group consisting of ethoxylated sorbitan esters like PEG-40 sorbitan diisostearate, polysorbate 80 (PS80), polysorbate 20 (PS20), polysorbate 40 (PS40), polysorbate 60 (PS60); bock co-polymers like poloxamer 124, poloxamer 188, poloxamer 331, poloxamer 407, fatty acids ethoxylates like PEG-5 oleate, PEG-8 stearate, polyoxyl 40 stearate, polyoxyl 15 hydroxystearate, fatty alcohol ethoxylates like steareth 40; fatty acid esters like ascorbyl palmitate, beeswax, polyglyceryl 3-oleate, propylene glycol monocaprylate, propylene glycol monolaurate; fatty alcohols like cetostearyl alcohol, cetyl alcohol, myristic alcohol, stearyl alcohol; glycerides; pegylated triglycerides; sugar esters and are preferably polysorbates, more preferably polysorbate 80 (PS80). Buffers which can be used in the method of the present invention are phosphate, piperazine-N,N'-bis(2-ethanesulfonic acid), 2-Hydroxy-3-morpholinopropanesulfonic acid, N,N-bis[2-hydroxyethyl]-2-aminoethanesulfonic acid), (3-(N-morpholino)propanesulfonic acid), 2-[[1,3-dihydroxy-2-(hydroxymethyl)propan-2-yl]amino]ethanesulfonic acid, 4-(2-hydroxyethyl)-1-piperazineethanesulfonic acid), 3-(N,N-Bis[2-hydroxyethyl]amino)-2-hydroxypropanesulfonic acid, N,N-Bis(2-hydroxyethyl)-3-amino-2-hydroxypropanesulfonic acid, N-[Tris(hydroxymethyl)methyl]glycine, Diglycine, 4-(2-Hydroxyethyl)-1-piperazinepropanesulfonic acid, N,N-Bis(2-hydroxyethyl)glycine, N-[Tris(hydroxymethyl)methyl]-3-aminopropanesulfonic acid, N-(1,1-Dimethyl-2-hydroxyethyl)-3-amino-2-hydroxypropanesulfonic acid.

In one embodiment, the agent which interacts with the lid domain of the lipase or a fragment thereof is added to a suspension of the solid carrier, preferably the agent which interacts with the lid domain of the lipase or a fragment thereof is added together with the lipase or a fragment thereof, the protease or a fragment thereof and the amylase or a fragment thereof to the suspension of the solid carrier prior to immobilization of the lipase or a fragment thereof, the protease or a fragment thereof and the amylase or a fragment thereof on the solid carrier. The lipase or a fragment thereof, the protease or a fragment thereof and the amylase or a fragment thereof can be added in form of pancreatin the suspension of the solid carrier prior to immobilization of the lipase or a fragment thereof, the protease or a fragment thereof and the amylase or a fragment thereof on the solid carrier

The immobilization of the lipase or a fragment thereof, the protease or a fragment thereof and the amylase or a fragment thereof on the solid carrier is usually carried out by adding a solution comprising a lipase or a fragment thereof, a protease or a fragment thereof and a amylase or a fragment thereof, or pancreatin or a solution thereof, to the suspension of the solid carrier. In a preferred embodiment the immobilization of the lipase or a fragment thereof, the protease or a fragment thereof and the amylase or a fragment thereof on the solid carrier is carried out by providing a suspension of the solid carrier and providing a solution comprising a lipase or a fragment thereof, a protease or a fragment thereof and a amylase or a fragment thereof, or pancreatin or a solution thereof,, wherein the suspension of the solid carrier is incubated with the solution comprising a lipase or a fragment thereof, a protease or a fragment thereof and a amylase or a fragment thereof, or pancreatin or a solution thereof, to allow the lipase or a fragment thereof, the protease or a fragment thereof and the amylase or a fragment thereof to bind on the surface of the solid carrier. In a more preferred embodiment the immobilization of a lipase on the solid carrier, a protease or a fragment thereof and a amylase or a fragment thereof, or pancreatin or a solution thereof, is carried out by providing a suspension of the solid carrier, providing a solution comprising a lipase or a fragment thereof, a protease or a fragment thereof and a amylase or a fragment thereof, or pancreatin or a solution thereof, and providing a solution of the agent which interacts with the lid domain of the lipase or a fragment thereof, wherein the suspension of the solid carrier is incubated with the solution comprising a lipase or a fragment thereof, a protease or a fragment thereof and a amylase or a fragment thereof, or pancreatin or a solution thereof, and with the solution of the agent which interacts with the lid domain of the lipase or a fragment thereof to allow the lipase or a fragment thereof, the protease or a fragment thereof and the amylase or a fragment thereof to bind on the surface of the solid carrier.

In one embodiment the surface of the solid carrier is modified to introduce a molecule or functional chemical group as anchoring point i.e. as anchoring point for the protein e.g. the lipase or a fragment thereof, the protease or a fragment thereof and the amylase or a fragment thereof or for the linker connecting the protein e.g. the lipase or a fragment thereof, the protease or a fragment thereof and the amylase or a fragment thereof to the solid carrier. Preferably, said anchoring point is an amine functional chemical group or moiety. As a non-limiting example, an amino-modified surface of the solid carrier e.g. an amino-modified silica surface may be used as modified solid carrier. Such an amino-modified surface of the solid carrier may be obtained by reacting a solid carrier having a silica surface with an amino silane, e.g. with APTES. Thus in a preferred embodiment, the solid carrier is a solid carrier having a silica surface with an amino-modified surface, more preferably a solid carrier obtained by reacting the solid carrier having a silica surface with an amino silane, e.g. with APTES. Such a modified carrier may form an amide linkage between the protein e.g. the lipase or a fragment thereof, the protease or a fragment thereof and the amylase or a fragment thereof and the amine group at the surface of the carrier material or an amide linkage between the linker and the amine group at the surface of the carrier material. In one embodiment the introduced molecule or functional chemical group as anchoring point is homogeneously distributed on the surface of the solid carrier.

In one embodiment the agent which interacts with the lid domain of the lipase or a fragment thereof is selected from the group consisting of a colipase or a fragment thereof, a colipase mimicking pepide, and an amphipathic molecule. Preferably, the agent which interacts with the lid domain of the lipase or a fragment thereof is selected from the group consisting of a colipase or a fragment thereof, a colipase mimicking pepide, and a bile salt, more preferably selected from the group consisting of a colipase or a fragment thereof, a colipase mimicking pepide, and sodium taurocholate , even more preferably selected from the group consisting of a colipase or a fragment thereof, a colipase mimicking pepide as shown in SEQ ID NO: 1, and sodium taurocholate. Most preferably the agent which interacts with the lid domain of the lipase or a fragment thereof is a bile salt, in particular sodium taurocholate.

In one embodiment the agent which interacts with the lid domain of the lipase or a fragment thereof interacts specifically with the lid domain of the lipase or a fragment thereof so that the lipase or a fragment thereof shifts to and/or maintains the open conformation.

In one embodiment from about 50% to 100%, preferably from about 80% to 100%, more preferably from about 90% to 100%, even more preferably about 100% of the lipase or a fragment thereof immobilized on the surface of the solid carrier is in the open conformation.

In some embodiments the protective layer has a defined thickness of about 1 to about 200 nm, usually 1 to about 100 nm, preferably about 1 to about 50nm, more preferably about 1 to about 25 nm, even more preferably about 1 to about 20 nm, in particular about 1 to about 15 nm. The most preferred defined thickness is about 1 to about 10 nm. In some embodiments the layer has a defined thickness of about 5 to about 100 nm, preferably about 5 to about 50 nm, more preferably about 5 to about 25 nm, even more preferably about 5 to about 20 nm, in particular about 5 to about 15 nm. The most preferred defined thickness is about 5 to about 10 nm. The protective layer is usually porous and the pore size is between 1 and 20 nm, preferably between 1 and 5 nm. The protective layer thickness can be measured, by using a microscope such as scanning electron microscope (SEM), transmission electron microscopy (TEM), scanning probe microscopy (SPM), light scattering methods or by ellipsometry.

In one embodiment, the protein or fragment thereof, e.g. the lipase or a fragment thereof, the protease or a fragment thereof and the amylase or a fragment thereof is partially embedded by the protective layer. In a preferred embodiment the protein or fragment thereof, e.g. the lipase or a fragment thereof, the protease or a fragment thereof and the amylase or a fragment thereof is at least partially embedded by the protective layer. In a more preferred embodiment the protein or fragment thereof, e.g. the lipase or a fragment thereof, the protease or a fragment thereof and the amylase or a fragment thereof is fully embedded by the protective layer.

In one embodiment, the protective layer embeds the solid carrier and embeds the protein or fragment thereof, e.g. the lipase or a fragment thereof, the protease or a fragment thereof and the amylase or a fragment thereof immobilized on the surface of the solid carrier. In one embodiment, the functional constituent immobilized on the surface of the protective layer, is not embedded by the protective layer. Preferably, the protective layer fully embeds the solid carrier and fully embeds the protein or fragment thereof, e.g. the lipase or a fragment thereof, the protease or a fragment thereof and the amylase or a fragment thereof immobilized on the surface of the solid carrier. More preferably, the protective layer fully embeds the solid carrier and fully embeds the protein or fragment thereof, e.g. the lipase or a fragment thereof, the protease or a fragment thereof and the amylase or a fragment thereof immobilized on the surface of the solid carrier and the functional constituent immobilized on the surface of the protective layer is not embedded by the protective layer. If the protective layer fully embeds the solid carrier and fully embeds the protein or fragment thereof, e.g. the lipase or a fragment thereof, the protease or a fragment thereof and the amylase or a fragment thereof immobilized on the surface of the solid carrier, the protein or fragment thereof, e.g. the lipase or a fragment thereof, the protease or a fragment thereof and the amylase or a fragment thereof is fully, i.e. 100% covered by the protective layer, i.e. that also the active site is covered and the solid carrier is fully, i.e. 100% covered by the protective layer.

In a preferred embodiment the lipase or a fragment thereof, the protease or a fragment thereof and the amylase or a fragment thereof used in the present invention are comprised by pancreatin. In a more preferred embodiment pancreatin is used to immobilize the lipase or a fragment thereof, the protease or a fragment thereof and the amylase or a fragment thereof on the surface of the solid carrier. Thus, the present invention also provides a nanoparticle composition comprising a) a nanoparticle comprising a solid carrier; pancreatin, wherein the pancreatin comprises a lipase or a fragment thereof wherein the lipase or a fragment thereof is in the open conformation, a protease or a fragment thereof and an amylase or a fragment thereof, wherein the lipase or a fragment thereof, the protease or a fragment thereof and the amylase or a fragment thereof are immobilized on the surface of the solid carrier; an agent which interacts with the lid domain of the lipase or a fragment thereof; a protective layer to protect the lipase or a fragment thereof, the protease or a fragment thereof and the amylase or a fragment thereof by embedding the lipase or a fragment thereof, the protease or a fragment thereof and the amylase or a fragment thereof; and a functional constituent immobilized on the surface of the protective layer, wherein the functional constituent immobilized on the surface of the protective layer is a polymer comprising repeat units wherein each repeat unit comprises at least one amino group and/or at least one thiol group; b) an amino acid; c) a non-reducing sugar and/or a non-reducing sugar alcohol; and d) a surfactant

The nanoparticle composition of the present invention is usually produced in a reaction vessel like a reactor. The formation of the protective layer is usually carried out by forming the respective protective layer by building blocks, wherein the building blocks build the protective layer in a polycondensation reaction. The polycondensation can be performed in different solvents, preferably in aqueous solution. Polycondensation can be easily controlled and stopped if appropriate, allowing achievement of a defined thickness of the protective layer. The choice of the building blocks, which can be used to build the protective layer, may depend on the known structure of the protein e.g. the lipase or a fragment thereof, the protease or a fragment thereof and the amylase or a fragment thereof in order to adapt the affinity of the protective layer according to optimal and/or desired parameters. As building blocks for the protective layer usually structural building blocks and protective building blocks are used to build the protective layer. Structural building blocks which can be used are e.g. tetraethylorthosilicate (designated herein as "TEOS" or "T"). Protective building blocks which can be used are e.g. 3-Aminopropyltriethoxysilane (designated herein as "APTES" or "A"), Propyltriethyoxysilane (designated herein as "PTES" or P"), Isobutyltriethoxysilane (designated as "IBTES"), Hydroxymethyltriethoxysilane (designated herein as "HTMEOS" or H), Benzyltriethoxysilane (designated herein as "BTES"), Ureidopropyltriethoxysilane (designated as "UPTES"), or Carboxyethyltriethoxysilane (designated herein as "CETES"). Structural building blocks are usually precursors of inorganic silica, capable of forming 4 covalent bonds in the layer formed. Protective building blocks are usually organosilanes, bearing an organic moiety endowed with the ability to interact with the proteins e.g. the lipase or a fragment thereof, the protease or a fragment thereof and the amylase or a fragment thereof. Preferred structural building blocks are tetravalent silanes, in particular tetra-alkoxy-silanes. Preferred protective building blocks are trivalent silanes, in particular tri-alkoxy-silanes. More preferred structural building blocks are mixtures of tetravalent silanes and trivalent silanes, in particular mixtures of tetra-alkoxy-silanes and tri-alkoxy-silanes. Even more preferred structural building blocks are selected from the group consisting of tetraethylorthosilicate, tetra-(2-hydroxyethyl)silane, and tetramethylorthosilicate. Even more preferred protective building blocks are selected from the group consisting of carboxyethylsilanetriol, benzylsilanes, propylsilanes, isobutylsilanes, n-octylsilanes, hydroxysilanes, bis(2-hydroxyethyl)-3 -aminopropylsilanes, aminopropylsilanes, ureidopropylsilanes, (N-Acetylglycyl)-3-aminopropylsilanes, hydroxy(polyethyleneoxy)propyl]triethoxysilanes, in particular selected from benzyltriethoxysilane (BTES), propyltriethoxysilane, isobutyltriethoxysilane, n-octyltriethoxysilane, hydroxymethyltriethoxysilane, bis(2-hydroxyethyl)-3 - aminopropyltriethoxysilane, 3-Aminopropyltriethoxysilane, ureidopropyltriethoxysilane, (N-Acetylglycyl)-3-aminopropyltriethoxysilane, or selected from benzyltrimethoxysflane, propyltrimethoxysilane, isobutylimethoxysilane, n-octyltrimethoxysilane, hydroxyrnethyltrimethoxysilane, bis(2-hydroxyethyl)-3 -aminopropyltrimethoxysilane, 3-Aminopropyltriethoxysilane, ureidopropyltriethoxysilane, (N-Acetylglycyl)-3-aminopropyltriethoxysilane, or selected from benzyltrimethoxysilane, propyltrimethoxysilane, isobutylimethoxysilane, n-octyltrimethoxysilane, hydroxymethyltrimethoxysilane, bis(2-hydroxyethyl)-3-aminopropyltrimethoxysilane, aminopropyltrimethoxysilane, ureidopropyltrimethoxysilane (N-Acetylglycyl)-3-aminopropyltrimethoxysilane or selected from benzyltrihydroxyethoxysilane, propyltrihydroxyethoxysilane, isobutyltrihydroxyethoxysilane, n-octyltrihydroxyethoxysilane, hydroxymefilyltrihydroxyethoxysilane, bis(2-hydroxyethyl)-3 - aminopropyltrihydroxyethoxysilane, aminopropyltrihydroxyethoxysilane, Ureidopropyltrihydroxyethoxysilane (N-Acetylglycyl)-3-aminopropyltrihydroxymethoxysilane. Particular preferred building blocks are TEOS as structural building block and APTES, BTES, and/or HTMEOS, preferably APTES and/or BTES as protective building block. In particular TEOS as structural building block and APTES and/or BTES as protective building block are used to build the protective layer.

The reaction time of the building blocks with the solid carrier carrying the immobilized enzymes can depend on the length of the linker, if a linker is used, and the size of the protein e.g. the lipase or a fragment thereof, the protease or a fragment thereof and the amylase or a fragment thereof. The reaction is usually carried out for a time period of between 0.5 to 10 hours, preferably between 1 and 5 hours, more preferably between 1 and 4 hours, even more preferably between 2 and 4 hours, preferably in aqueous solution and preferably at room temperature of about 5 to about 25 °C or at about 20 °C. The formation of the protective layer can be stopped by actively stopping the polycondensation reaction e.g. by removing the non-reacted building blocks e.g. by a washing step or by self-stopping of the polycondensation reaction caused by a limited amount of buidling blocks.

In a further more preferred embodiment the protein e.g. the lipase or a fragment thereof, the protease or a fragment thereof and the amylase or a fragment thereofis immobilized on the solid carrier by at least partly modifying the surface of the solid carrier by introducing a molecule as anchoring point as described supra for the protein e.g. the lipase or a fragment thereof, the protease or a fragment thereof and the amylase or a fragment thereofand by using a linker, preferably a cross-linker binding to the anchoring point and the protein e.g. the lipase or a fragment thereof, the protease or a fragment thereof and the amylase or a fragment thereof.

In one embodiment the introduced molecule as anchoring point and/or the linker are homogeneously distributed on the surface of the solid carrier.

In a preferred embodiment the cross-linker is selected from the group consisting of glutaraldehyde, disuccinimidyl tartrate, bis[sulfosuccinimidyl]suberate, ethylene glycolbis(sulfosuccinimidylsuccinate), dimethyl adipimidate, dimethyl pimelimidate, sulfosuccinimidyl (4-iodoacetyl) aminobenzoate, 1,5-difluoro-2,4-dinitrobenzene, activated sulfhydrils, sulfhydryl-reactive 2-pyridyldithiol, BSOCOES (Bis[2-(succinimidooxycarbonyloxy)ethyl]sulfone), DSP (Dithiobis[succinimidyl]propionate]), DTSSP (3,3 '-Dithiobis[sulfosuccinimidyl]propionate]), DTBP (Dimethyl 3,3 '-dithiobispropionimidate 2 HCl), DST (Disuccinimidyl tartarate), Sulfo-LC-SMPT (4-Sulfosuccinimidyl-6-methyl-a-(2-pyridyldithio)toluamido]hexanoate)), SPDP (N-Succinimidyl 3-(2-pyridyldithio)-propionate), LC-SPDP (Succinimidyl 6-(3-[2-pyridyldithio]-propionamido)hexanoate), SMPT (4-Succinimidyloxycarbonyl-methyl-a-[2-pyridyldithio]toluene), DPDPB (1,4-Di-[3'-(2'-pyridyldithio)-propionamido]butane), DTME (Dithio-bismaleimidoethane), BMDB (1,4 bismaleimidyl-2,3-dihydroxybutane). More preferably said cross-linker is selected from glutaraldehyde, disuccinimidyl tartrate, disuccinimidyl suberate, bis[sulfosuccinimidyl] suberate, ethylene glycolbis(sulfosuccinimidylsuccinate), dimethyl adipimidate, dimethyl pimelimidate, sulfosuccinimidyl (4-iodoacetyl) aminobenzoate, 1,5-difluoro-2,4-dinitrobenzene, activated sulfhydrils (e.g. suflhydryl-reactive 2-pyridyldithio) and a colipase-mimicking peptide, wherein the colipase-mimicking peptide can be functionalized with a chemical group that enable covalent binding to the solid carrier surface. In a more preferred embodiment the cross-linker is selected from the group consisting of glutaraldehyde, disuccinimidyl tartrate, bis[sulfosuccinimidyl]suberate, ethylene glycolbis(sulfosuccinimidylsuccinate), dimethyl adipimidate, dimethyl pimelimidate, sulfosuccinimidyl (4-iodoacetyl) aminobenzoate, 1,5-difluoro-2,4-dinitrobenzene, BSOCOES (Bis[2-(succinimidooxycarbonyloxy)ethyl]sulfone), DSP (Dithiobis[succinimidyl]propionate]), DTSSP (3,3 '-Dithiobis[sulfosuccinimidyl]propionate]), DTBP (Dimethyl 3,3 '-dithiobispropionimidate·2 HCl), DST (Disuccinimidyl tartarate), BMDB (1,4 bismaleimidyl-2,3-dihydroxybutane). More preferably said cross-linker is selected from glutaraldehyde, disuccinimidyl tartrate, disuccinimidyl suberate, bis[sulfosuccinimidyl] suberate, ethylene glycolbis(sulfosuccinimidylsuccinate), dimethyl adipimidate, dimethyl pimelimidate, sulfosuccinimidyl (4-iodoacetyl) aminobenzoate, 1,5-difluoro-2,4-dinitrobenzene, activated sulfhydrils (e.g. suflhydryl-reactive 2-pyridyldithio). Most preferred is glutaraldehyde.

After the protective layer has been formed, the suspension obtained can be washed to remove excess agent which interacts with the lid domain of the lipase or a fragment thereof. In one embodiment, after the protective layer has been formed, the solid carrier comprising the protein e.g. the lipase or a fragment thereof, the protease or a fragment thereof and the amylase or a fragment thereofand the protective layer can be stored. Storing is usually accomplished e.g. by washing the composition formed e.g. with a buffer and storing it suspended or solved in that buffer for a desired time period. In a preferred embodiment the solid carrier comprising the protein e.g. the lipase or a fragment thereof, the protease or a fragment thereof and the amylase or a fragment thereofand the protective layer is stored at a constant temperature between 2 to 25 °C. In a further preferred embodiment, the solid carrier comprising protein e.g. the lipase or a fragment thereof, the protease or a fragment thereof and the amylase or a fragment thereofand the protective layer is stored 5 to 48 hours, preferably 10 to 30 hours. More preferably the solid carrier comprising the protein e.g. the lipase or a fragment thereof, the protease or a fragment thereof and the amylase or a fragment thereofand the protective layer is stored at a constant temperature between 2 to 25 °C, preferably at room temperature for 10 to 30 hours.

In one embodiment, the functional constituent binds to mucus.

In one embodiment a polymer comprising repeat units wherein each repeat unit comprises at least one amino group and/or at least one thiol group is a polymer comprising repeat units wherein each repeat unit comprises at least one amino group.

In one embodiment a polymer comprising repeat units wherein each repeat unit comprises at least one amino group and/or at least one thiol group is a polymer comprising repeat units wherein each repeat unit comprises at least one thiol group.

In one embodiment a polymer comprising repeat units wherein each repeat unit comprises at least one amino group and/or at least one thiol group is selected from the group consisting of a polyglucosamin, a polymerized silane-PEG-NH2 and a polymerized silane comprising an amino group. In a preferred embodiment the polymer comprising repeat units wherein each repeat unit comprises at least one amino group and/or at least one thiol group, is selected from the group consisting of a polyglucosamin, a polymerized silane-PEG-NH2 and polymerized APTES.

In a more preferred embodiment the polymer comprising repeat units wherein each repeat unit comprises at least one amino group and/or at least one thiol group is selected from the group consisting of a polyglucosamin selected from the group consisting of chitin, chitosan, polyglucosaminoglycans, chondroitin, heparin, keratan and dermatan or a derivative thereof; a polymerized silane-PEG-NH2; and a polymerized silane comprising an amino group, preferably a polymerized APTES. In an even more preferred embodiment the polymer comprising repeat units wherein each repeat unit comprises at least one amino group and/or at least one thiol group, is a polyglucosamin, preferably a polyglucosamin selected from the group consisting of chitin, chitosan, polyglucosaminoglycans, chondroitin, heparin, keratan and dermatan or a derivative thereof, more preferably a chitosan or a derivative thereof.

A preferred polyglucosamin of the present invention is selected from the group consisting of chitin, chitosan, polyglucosaminoglycans, chondroitin, heparin, keratan and dermatan or a derivative thereof. Most preferred is a chitosan or a derivative thereof.

A preferred silane-PEG-NH2 of the polymerized silane-PEG-NH2 is selected from the group consisting of silane-PEG4-NH2, silane-PEG2000-NH2, and silane-PEG5000-NH2. A preferred polymerized silane comprising an amino group is selected from the group consisting of APTES, amino-butyl-TES, amino-pentyl-TES, amino-hexyl-TES, amino-heptyl-TES, and amino-octyl-TES, and is in particular APTES.

In a further embodiment a polymer comprising repeat units wherein each repeat unit comprises at least one amino group and/or at least one thiol group is selected from the group consisting of a polyglucosamin, a polymerized silane-PEG-NH2, a polymerized silane comprising an amino group, a polymerized silane comprising a thiol group, a polycarbophil-cysteine conjugate, a polymerized silane-PEG-thiol and a polycysteine. In a further more preferred embodiment the polymer comprising repeat units wherein each repeat unit comprises at least one amino group and/or at least one thiol group is selected from the group consisting of a polyglucosamin selected from the group consisting of chitin, chitosan, polyglucosaminoglycans, chondroitin, heparin, keratan and dermatan or a derivative thereof; a polymerized silane-PEG-NH2; a polymerized silane comprising a thiol group, preferably a polymerized MPTS; a polycarbophil-cysteine conjugate; a polymerized silane-PEG-thiol; and a polycysteine. In an even more preferred embodiment the polymer comprising repeat units wherein each repeat unit comprises at least one amino group and/or at least one thiol group, is a polyglucosamin or a polymerized silane comprising a thiol group, preferably a polyglucosamin selected from the group consisting of chitin, chitosan, polyglucosaminoglycans, chondroitin, heparin, keratan and dermatan or a derivative thereof, more preferably a chitosan or a derivative thereof or a polymerized silane comprising a thiol group, a polycarbophil-cysteine conjugate, and a polymerized silane-PEG-thiol, preferably a polymerized silane comprising a thiol group.

In a particular embodiment, the polymer comprising repeat units wherein each repeat unit comprises at least one amino group and/or at least one thiol group, is selected from the group consisting of chitin, chitosan, polyglucosaminoglycans, chondroitin, heparin, keratin, dermatan or a derivative thereof in particular chitosan or a derivative thereof, a polymerized silane-PEG-NH2 selected from the group consisting of polymerized silane-PEG4-NH2, polymerized silane-PEG2000-NH2, polymerized silane-PEG5000-NH2, a polymerized silane comprising an amino group which is preferably polymerized APTES and a polymerized silane comprising a thiol group, which is preferably polymerized MPTS.

In one embodiment a polymer comprising repeat units wherein each repeat unit comprises at least one amino group and/or at least one thiol group is selected from the group consisting of a polyglucosamin, a polymerized silane-PEG-NH2, a polymerized silane comprising an amino group and a polymerized silane comprising a thiol group. In a preferred embodiment the polymer comprising repeat units wherein each repeat unit comprises at least one amino group and/or at least one thiol group, is selected from the group consisting of a polyglucosamin, a polymerized silane-PEG-NH2, polymerized APTES and polymerized MPTS.

In a more preferred embodiment the polymer comprising repeat units wherein each repeat unit comprises at least one amino group and/or at least one thiol group is selected from the group consisting of a polyglucosamin selected from the group consisting of chitin, chitosan, polyglucosaminoglycans, chondroitin, heparin, keratan and dermatan or a derivative thereof; a polymerized silane-PEG-NH2; a polymerized silane comprising an amino group, preferably a polymerized APTES; and a polymerized silane comprising a thiol group, preferably polymerized MPTS. In a particular embodiment, the polymer comprising repeat units wherein each repeat unit comprises at least one amino group and/or at least one thiol group, is selected from the group consisting of chitin, chitosan, polyglucosaminoglycans, chondroitin, heparin, keratin, dermatan or a derivative thereof, in particular chitosan or a derivative thereof, a polymerized silane-PEG-NH2 selected from the group consisting of polymerized silane-PEG4-NH2, polymerized silane-PEG2000-NH2, polymerized silane-PEG5000-NH2, a polymerized silane comprising an amino group which is APTES and a polymerized silane comprising an thiol group which is MPTS.

In one embodiment a polymer comprising repeat units wherein each repeat unit comprises at least one thiol group is selected from the group consisting of a polymerized silane comprising a thiol group, a polycarbophil-cysteine conjugate, a polymerized silane-PEG-thiol and a polycysteine, and is preferably selected from the group consisting of a polymerized silane comprising a thiol group, a polycarbophil-cysteine conjugate, and a polymerized silane-PEG-thiol, and is more preferably a polymerized silane comprising a thiol group, and is most perferably polymerized MPTS. In one embodiment a polymerized silane comprising a thiol group is preferably polymerized MPTS.

In one embodiment 5% to 100%, preferably 10% to 100%, more preferably 50% to 100%, of the surface of the protective layer is covered with a polymer comprising repeat units wherein each repeat unit comprises at least one amino group and/or at least one thiol group.

In one embodiment the functional constituent is immobilized on the surface of the protective layer by binding, preferably covalent binding. In a preferred embodiment the functional constituent is immobilized on the surface of the protective layer by non-covalent binding, preferably by electrostatic interactions. In a more preferred embodiment, the polymer comprising repeat units wherein each repeat unit comprises at least one amino group and/or at least one thiol group is immobilized on the surface of the protective layer by covalent binding.

In one embodiment the functional constituent is immobilized on the surface of the protective layer using a spacer binding to the surface of the protective layer and the functional constituent. Thus in one embodiment the present invention comprises a nanoparticle composition comprising a) a nanoparticle comprising a solid carrier, a protein or a fragment thereof immobilized on the surface of the solid carrier, a protective layer to protect the protein or a fragment thereof by embedding the protein or a fragment thereof, and a functional constituent immobilized on the surface of the protective layer, wherein the functional constituent immobilized on the surface of the protective layer is a polymer comprising repeat units wherein each repeat unit comprises at least one amino group and/or at least one thiol group; b) an amino acid; c) a non-reducing sugar and/or a non-reducing sugar alcohol; and d) a surfactant, wherein the functional constituent is immobilized on the surface of the protective layer by a spacer. Examples of such a spacer include a polyethylene such as PEG4, PEG2000, PEG5000. A functional constituent immobilized on the surface of the protective layer, by a spacer is usually produced by firstly reacting the spacer with the functional constituent, so that the spacer binds to the functional constituent and then the functional constituent bound to the spacer is reacted with the the surface of the protective layer.

The immobilization of the functional constituent to the surface of the protective layer is usually carried out in a reaction vessel like a reactor by suspending the solid carrier carrying the protein e.g. the enzyme embedded in a protective layer as described supra in e.g. in water, buffer or non-ionic surfactants or mixtures thereof, preferably in mixtures of water and non-ionic surfactants. Non-ionic surfactants are usually selected from the group consisting of ethoxylated sorbitan esters like PEG-40 sorbitan diisostearate, polysorbate 80 (PS80), polysorbate 20 (PS20), polysorbate 40 (PS40), polysorbate 60 (PS60); bock co-polymers like poloxamer 124, poloxamer 188, poloxamer 331, poloxamer 407, fatty acids ethoxylates like PEG-5 oleate, PEG-8 stearate, polyoxyl 40 stearate, polyoxyl 15 hydroxystearate, fatty alcohol ethoxylates like steareth 40; fatty acid esters like ascorbyl palmitate, beeswax, polyglyceryl 3-oleate, propylene glycol monocaprylate, propylene glycol monolaurate; fatty alcohols like cetostearyl alcohol, cetyl alcohol, myristic alcohol, stearyl alcohol; glycerides; pegylated triglycerides; sugar esters and are preferably polysorbates, more preferably polysorbate 80 (PS80). The functional component is then added to the suspension to react usually under stirring with the surface of the protetctive layer to immobilize the functional constitutent on the surface of the protective layer. Ususally such obtained composition is washed and resuspended into water, buffer or non-ionic surfactants or mixtures thereof. Immobilization takes place by non-covalent binding e.g. electrostatic binding or by covalent binding of the functional constituent. The functional constituent may be immobilized by chemically modifying the surface of the protective layer and the functional constituent using e.g. "click chemistry" such as copper-catalyzed click chemistry (Copper-catalysed azide-alkyne cycloaddition, see e.g. Kolb et al. (2001) Angew. Chem. 40(11)2004-2021) or by copper free click chemistry (Wittig G, A Chem Ber, 1961, 94, 3260), e.g. the solid carrier carrying the protein e.g. the enzyme embedded in a protetctive layer as described supra is first reacted with a reactive compound like an ethynyl compound and the functional constituent is modified by adding a reactive compound e.g.an azide residue and then both components are reacted to immobilize the functional constituent on the surface of the protective layer.

In one embodiment the amino acid of b) is selected from the group consisting of alanine, arginine, cysteine, glutamic acid, glutamine, glycine, histidine, isoleucine, leucine, lysine, methionine, phenylalanine, serine, and tryptophan. In a preferred embodiment the amino acid of b) is histidine and cysteine; histidine and tryptophan; histidine and methionine; glycine and cysteine; glycine and tryptophan; glycine and methionine; arginine and cysteine; arginine and tryptophan; arginine and methionine; lysine and cysteine; lysine and tryptophan; or lysine and methionine, preferably histidine and cysteine or lysine and tryptophan.

In one embodiment the non-reducing sugar and/or non-reducing sugar alcohol of c) is selected from the group consisting of glucose, galactose, lactose, sucrose, melbiose, trehalose, mannitol, sorbitol, erythritol, glucosamine, myo-inositol, xylitol, cyclodextrins, stachyose, lactosucrose, melezidose, raffinose, alginate, carrageenan, chitosan, chondroitin sulfate, dextran, inulin, ficoll, hyaluronan, and carboxymethylcellulose. In a preferred embodiment the non-reducing sugar and/or non-reducing sugar alcohol of c) is selected from the group consisting of trehalose, sucrose, maltodextrin and mannitol, more preferably from trehalose, sucrose, and maltodextrin.

In one embodiment the surfactant is selected from the group consisting of Polysorbate 80, Polysorbate 20, Brij 97, Kolliphor EL, Poloxamer 407, Poloxamer 188, Sodium dodecyl sulfate, Sodium taurocholate, Tyloxapol, Polyvinylpyrrolidone 10K, Polyvinylpyrrolidone 40K.

In a preferred embodiment the surfactant of d) is a non-ionic surfactant, more preferably a non-ionic surfactant selected from the group consisting of polyvinylpyrrolidone 10K (PVP 10K), polyvinylpyrrolidone 40K (PVP 40K) and polysorbate 80 (PS80), even more preferably polysorbate 80 (PS80).

In one embodiment the amino acid of b) is selected from the group consisting of alanine, arginine, cysteine, glutamic acid, glutamine, glycine, histidine, isoleucine, leucine, lysine, methionine, phenylalanine, serine, and tryptophan; the non-reducing sugar and/or non-reducing sugar alcohol of c) is selected from the group consisting of glucose, galactose, lactose, sucrose, melbiose, trehalose, mannitol, sorbitol, erythritol, glucosamine, myo-inositol, xylitol, cyclodextrins, stachyose, lactosucrose, melezidose, raffinose, alginate, carrageenan, chitosan, chondroitin sulfate, dextran, inulin, ficoll, hyaluronan, and carboxymethylcellulose; and/or the surfactant of d) is a non-ionic surfactant, preferably a non-ionic surfactant is selected from the group consisting of polyvinylpyrrolidone 10K (PVP 10K), polyvinylpyrrolidone 40K (PVP 40K) and polysorbate 80 (PS80).

In a preferred embodiment the amino acid of b) is histidine and cysteine; histidine and tryptophan; histidine and methionine; glycine and cysteine; glycine and tryptophan; glycine and methionine; arginine and cysteine; arginine and tryptophan; arginine and methionine; lysine and cysteine; lysine and tryptophan; or lysine and methionine, preferably histidine and cysteine or lysine and tryptophan; the non-reducing sugar and/or non-reducing sugar alcohol of c) is selected from the group consisting of trehalose, sucrose, maltodextrin and mannitol; and the surfactant of d) is selected from the group consisting of polyvinylpyrrolidone 10K (PVP 10K), polyvinylpyrrolidone 40K (PVP 40K) and polysorbate 80 (PS80).

In the most preferred embodiment the amino acid of b) are cysteine and histidine; the non-reducing sugar and/or non-reducing sugar alcohol of c) is trehalose; and the surfactant of d) is polysorbate 80.

A preferred nanoparticle composition comprises or contains:
a) a nanoparticle comprising a solid carrier, whereby the solid carrier is a silica nanoparticle (SNP), a lipase or a fragment thereof immobilized on the surface of the solid carrier wherein the lipase or a fragment thereof is in the open conformation, a protease or a fragment thereof immobilized on the surface of the solid carrier, an amylase or a fragment thereof immobilized on the surface of the solid carrier, an agent which interacts with the lid domain of the lipase or a fragment thereof wherein the gent which interacts with the lid domain of the lipase or a fragment thereof is a bile salt, in particular sodium taurocholate, a protective layer to protect the lipase or a fragment thereof, the protease or a fragment thereof and the amylase or a fragment thereof by embedding the lipase or a fragment thereof, the protease or a fragment thereof and the amylase or a fragment thereof, wherein the protective layer comprises tetravalent silanes and trivalent silanes;
and a functional constituent immobilized on the surface of the protective layer, wherein the functional constituent immobilized on the surface of the protective layer is a polymer comprising repeat units wherein each repeat unit comprises at least one amino group, preferably wherein the functional constituent immobilized on the surface of the protective layer is chitosan;
b) an amino acid wherein the amino acid is cysteine and histidine; c) a non-reducing sugar and/or non-reducing sugar alcohol wherein the non-reducing sugar and/or non-reducing sugar alcohol is trehalose; and d) a surfactant wherein the surfactant is polysorbate 80.

In more preferred embodiment the nanoparticle of a) amounts from 48 percent to 81 percent by weight based on total weight of the composition, the amino acid of b) amounts from 13 percent to 32 percent by weight based on total weight of the composition, the non-reducing sugar of c) amounts from 5 percent to 12 percent by weight based on total weight of the composition and the surfactant of d) amounts from 0.005 percent to 0.02 percent by weight based on total weight of the composition, provided that the combined weight percentage of all components of the composition does not exceed 100 weight percentage of the composition.

A more preferred nanoparticle composition comprises or contains:
a) a nanoparticle comprising a solid carrier, whereby the solid carrier is a silica nanoparticle (SNP), a lipase or a fragment thereof immobilized on the surface of the solid carrier wherein the lipase or a fragment thereof is in the open conformation, a protease or a fragment thereof immobilized on the surface of the solid carrier, an amylase or a fragment thereof immobilized on the surface of the solid carrier, an agent which interacts with the lid domain of the lipase or a fragment thereof wherein the gent which interacts with the lid domain of the lipase or a fragment thereof is a bile salt, in particular sodium taurocholate, a protective layer to protect the lipase or a fragment thereof, the protease or a fragment thereof and the amylase or a fragment thereof by embedding the lipase or a fragment thereof, the protease or a fragment thereof and the amylase or a fragment thereof, wherein the protective layer comprises tetravalent silanes and trivalent silanes;
and a functional constituent immobilized on the surface of the protective layer, wherein the functional constituent immobilized on the surface of the protective layer is a polymer comprising repeat units wherein each repeat unit comprises at least one amino group, preferably wherein the functional constituent immobilized on the surface of the protective layer is chitosan;
b) an amino acid wherein the amino acid is cysteine and histidine; c) a non-reducing sugar and/or non-reducing sugar alcohol wherein the non-reducing sugar and/or non-reducing sugar alcohol is trehalose; and d)a surfactant wherein the surfactant is polysorbate 80, wherein the nanoparticle of a) amounts from 48 percent to 81 percent by weight based on total weight of the composition, the amino acid of b) amounts from 13 percent to 32 percent by weight based on total weight of the composition, the non-reducing sugar of c) amounts from 5 percent to 12 percent by weight based on total weight of the composition and the surfactant of d) amounts from 0.005 percent to 0.02 percent by weight based on total weight of the composition, provided that the combined weight percentage of all components of the composition does not exceed 100 weight percentage of the composition.

In the most preferred embodiment in the nanoparticle composition, in particular in the solid nanoparticle composition, the nanoparticle of a) amounts to 63.99 percent by weight based on total weight of the composition, the amino acid of b) amounts to 27 percent by weight based on total weight of the composition, the non-reducing sugar of c) amounts to 9 percent by weight based on total weight of the composition and the surfactant of d) amounts to 0.01 percent by weight based on total weight of the composition.

A most preferred nanoparticle composition comprises or contains:
a) a nanoparticle comprising a solid carrier, whereby the solid carrier is a silica nanoparticle (SNP), a lipase or a fragment thereof immobilized on the surface of the solid carrier wherein the lipase or a fragment thereof is in the open conformation, a protease or a fragment thereof immobilized on the surface of the solid carrier, an amylase or a fragment thereof immobilized on the surface of the solid carrier, an agent which interacts with the lid domain of the lipase or a fragment thereof wherein the gent which interacts with the lid domain of the lipase or a fragment thereof is a bile salt, in particular sodium taurocholate, a protective layer to protect the lipase or a fragment thereof, the protease or a fragment thereof and the amylase or a fragment thereof by embedding the lipase or a fragment thereof, the protease or a fragment thereof and the amylase or a fragment thereof, wherein the protective layer comprises tetravalent silanes and trivalent silanes;
and a functional constituent immobilized on the surface of the protective layer, wherein the functional constituent immobilized on the surface of the protective layer is a polymer comprising repeat units wherein each repeat unit comprises at least one amino group, preferably wherein the functional constituent immobilized on the surface of the protective layer is chitosan;
b) an amino acid wherein the amino acid is cysteine and histidine; c) a non-reducing sugar and/or non-reducing sugar alcohol wherein the non-reducing sugar and/or non-reducing sugar alcohol is trehalose; and d)a surfactant wherein the surfactant is polysorbate 80, wherein the nanoparticle of a) amounts to 63.99 percent by weight based on total weight of the composition, the amino acid of b) amounts to 27 percent by weight based on total weight of the composition, the non-reducing sugar of c) amounts to 9 percent by weight based on total weight of the composition and the surfactant of d) amounts to 0.01 percent by weight based on total weight of the composition,

In a preferred embodiment the nanoparticle composition is a solid composition, preferably a lyophilized composition.

In a more preferred embodiment the nanoparticle composition is in the form of capsules, preferably in the form of enteric-coated capsules. In an even more preferred embodiment the capsules comprise a lyophilized nanoparticle composition of the present invention. Capsules useful in the present invention can be made from polymers such as acrylic and methacrylic acid polymers, and other conventional acid insoluble polymers, e.g., methyl acrylate-methacrylic acid copolymers. Other polymers include, without limitation, cellulose acetate succinate, cellulose acetate phthalate (CAP), cellulose acetate butyrate, hydroxypropyl methyl cellulose, hydroxypropyl methyl cellulose phthalate (HPMCP), hydroxy propyl methyl cellulose acetate succinate (HPMCAS, hypermellose acetate succinate), cellulose acetate trimellitate (CAT), polyvinyl acetate phthalate (PVAP), methacrylic acid copolymers, algenic acid salts such as sodium alginate and potassium alginate, stearic acid, zein, and shellac. The capsules of the present invention are preferably made from hydroxypropyl methyl cellulose. As used herein, the term "enteric-coated capsules" refer to capsules which have enteric properties once the fill material e.g. the nanoparticle of the present invention is filled into the capsule, As used herein, the term "enteric" is used to refer to the dissolution or disintegration resistant property of a substance such that dissolution or disintegration does not occur in a gastric environment. For example, the capsules described herein include an enteric shell composition that dissolves in biological, artificial or simulated intestinal fluid rather than in biological, artificial or simulated gastric fluid. Enteric-coated capsules which can be used in the present invention are e.g. EUDRACAP^{®} capsules, in particular Eudragit^{®} L 30 D-55 coated HPMC capsules, commercialized by Evonik, which are preferred.

In one embodiment the nanoparticle composition is a liquid composition, preferably a liquid composition wherein the nanoparticles are in suspension.

In a further aspect the present invention provides the nanoparticle composition as described supra for use as a medicament.

In a further aspect the present invention provides the nanoparticle composition for use in a method of enzyme replacement therapy (ERT), preferably gastrointestinal enzyme replacement therapy, or for use in a method for the prevention, delay of progression or treatment of exocrine pancreatic insufficiency (EPI).

In a preferred embodiment the present invention provides the nanoparticle composition for use in a method for the prevention, delay of progression or treatment of exocrine pancreatic insufficiency (EPI). In a further preferred embodiment the present invention provides the nanoparticle composition for use in a method of enzyme replacement therapy (ERT), preferably gastrointestinal enzyme replacement therapy.

Also provided is the use of the nanoparticle composition as described herein for the manufacture of a medicament for the prevention, delay of progression or treatment of exocrine pancreatic insufficiency (EPI) in a subject. Also provided is the use of the nanoparticle composition as described herein for the prevention, delay of progression or treatment of exocrine pancreatic insufficiency (EPI) in a subject. Also provided is a method for the prevention, delay of progression or treatment of exocrine pancreatic insufficiency (EPI) in a subject, comprising administering to said subject a therapeutically effective amount of the nanoparticle composition as described herein. Also provided herein is the use of the nanoparticle composition as described herein for the manufacture of a medicament for a method of enzyme replacement therapy (ERT), preferably gastrointestinal enzyme replacement therapy. Also provided is the use of the nanoparticle composition as described herein in a method of enzyme replacement therapy (ERT), preferably gastrointestinal enzyme replacement therapy in a subject. Also provided is a method of enzyme replacement therapy (ERT), preferably gastrointestinal enzyme replacement therapy, in a subject, comprising administering to said subject a therapeutically effective amount of the nanoparticle composition as described herein.

A nanoparticle composition according to the invention is preferably a pharmaceutical composition and comprises a therapeutically effective amount of the composition as described herein and one or more suitable pharmaceutically acceptable carrier. A pharmaceutical composition according to the invention is suitable for oral administration to a subject. If not indicated otherwise, a pharmaceutical composition according to the invention is prepared in a manner known per se.

The nanoparticle composition, e.g. the pharmaceutical composition of the invention is administered preferably orally. The nanoparticle composition, e.g. the pharmaceutical composition of the invention may be administered according for a continuous period of one week or a part thereof, for two weeks, for three weeks for four weeks, for five weeks or for six weeks and then stopped for a period of one week, or a part thereof, for two weeks, for three weeks, for four weeks, for five weeks, or for six weeks or longer. The nanoparticle composition, e.g. the pharmaceutical composition of the present invention may conveniently be administered in unit dosage forms.

The expression "effective amount" or "therapeutically effective amount" as used herein refers to an amount capable of invoking one or more of the desired effects in a subject receiving the nanoparticle composition of the present invention. Determination of a therapeutically effective amount is well within the capability of those skilled in the art, especially in light of the detailed disclosure provided herein.

The terms "treatment"/"treating" as used herein includes: (1) delaying the appearance of clinical symptoms of the state, disorder or condition developing in an animal, particularly a mammal and especially a human, that may be afflicted with or predisposed to the state, disorder or condition but does not yet experience or display clinical or subclinical symptoms of the state, disorder or condition; (2) inhibiting the state, disorder or condition (e.g. arresting, reducing or delaying the development of the disease, or a relapse thereof in case of maintenance treatment, of at least one clinical or subclinical symptom thereof); and/or (3) relieving the condition (i.e. causing regression of the state, disorder or condition or at least one of its clinical or subclinical symptoms). The benefit to a patient to be treated is either statistically significant or at least perceptible to the patient or to the physician. However, it will be appreciated that when a medicament is administered to a patient to treat a disease, the outcome may not always be effective treatment.

As used herein, "delay of progression" means increasing the time to appearance of a symptom of or slowing the increase in severity of a symptom. Further, "delay of progression" as used herein includes reversing or inhibition of disease progression. "Inhibition" of disease progression or disease complication in a subject means preventing or reducing the disease progression and/or disease complication in the subject.

Preventive treatments comprise prophylactic treatments. In preventive applications, the pharmaceutical combination of the invention is administered to a subject suspected of having, or at risk for developing the above mentioned diseases or disorders. In therapeutic applications, the pharmaceutical combination is administered to a subject such as a patient already suffering from the above mentioned diseases or disorders, in an amount sufficient to cure or at least partially arrest the symptoms of the disease. Amounts effective for this use will depend on the severity and course of the disease, previous therapy, the subject's health status and response to the drugs, and the judgment of the treating physician.

In the case wherein the subject's condition does not improve, the pharmaceutical combination of the invention may be administered chronically, which is, for an extended period of time, including throughout the duration of the subject's life in order to ameliorate or otherwise control or limit the symptoms of the subject's disease or condition.

In the case wherein the subject's status does improve, the pharmaceutical combination may be administered continuously; alternatively, the dose of drugs being administered may be temporarily reduced or temporarily suspended for a certain length of time (i.e., a "drug holiday"). Once improvement of the patient's condition has occurred, a maintenance dose of the pharmaceutical combination of the invention is administered if necessary. Subsequently, the dosage or the frequency of administration, or both, is optionally reduced, as a function of the symptoms, to a level at which the improved disease is retained.

In a further aspect the present invention provides a method of producing a nanoparticle composition comprising a) a nanoparticle comprising a solid carrier, a lipase or a fragment thereof immobilized on the surface of the solid carrier wherein the lipase or a fragment thereof is in the open conformation, a protease or a fragment thereof immobilized on the surface of the solid carrier, an amylase or a fragment thereof immobilized on the surface of the solid carrier, an agent which interacts with the lid domain of the lipase or a fragment thereof, a protective layer to protect the lipase or a fragment thereof, the protease or a fragment thereof and the amylase or a fragment thereof by embedding the lipase or a fragment thereof, the protease or a fragment thereof and the amylase or a fragment thereof, and a functional constituent immobilized on the surface of the protective layer, wherein the functional constituent immobilized on the surface of the protective layer is a polymer comprising repeat units wherein each repeat unit comprises at least one amino group and/or at least one thiol group; b) an amino acid; c) a non-reducing sugar and/or a non-reducing sugar alcohol; and d) a surfactant; the method comprising the following steps:
(a) providing a solid carrier;
(b) providing a lipase or a fragment thereof, a protease or a fragment thereof and an amylase or a fragment thereof;
(c) providing an agent which interacts with the lid domain of a lipase or a fragment thereof;
(d) allowing the lipase or a fragment thereof of (b) to interact with the agent of (c);
(e) immobilizing the lipase or a fragment thereof, the protease or a fragment thereof and the amylase or a fragment thereof on the solid carrier;
(f) forming a protective layer on the surface of the solid carrier to protect the lipase or a fragment thereof, the protease or a fragment thereof and the amylase or a fragment thereof immobilized on the solid carrier;
(g) immobilizing a functional constituent on the surface of the protective layer to obtain the nanopaticle, wherein the functional constituent immobilized on the surface of the protective layer is a polymer comprising repeat units, wherein each repeat unit comprises at least one amino group and/or at least one thiol group;
(h) providing the nanoparticles obtained in (g) in suspension and adding the amino acid of b); the non-reducing sugar and/or the non-reducing sugar alcohol of c) and the surfactant of d); and optionally;
.(j) lyophilizing the supspension to obtain dry pellets and further optionally blending the dry pellets to obtain a dry powder; and optionally
(k) loading the dry powder into capsules, preferably enterically coated capsules.

In a preferred embodiment, pancreatin comprising a lipase or a fragment thereof, a protease or a fragment thereof and an amylase or a fragment thereof is provided in step b).

Step (a) is usually carried out by providing the solid carrier in suspension in water or a buffer, preferably in water, non-ionic surfactants or a buffer or mixtures thereof, preferably in buffer, more preferably in suspension in water and/or non-ionic surfactants, even more preferably in suspension in water and/or non-ionic surfactants wherein no buffer is present in the suspension, in particular in suspension in mixtures of water and non-ionic surfactants, more particular in suspension in mixtures of water and non-ionic surfactants wherein no buffer is present in the suspension. In a preferred embodiment, pancreatin is provided in step b). Step b) and c) are usually performed separately or can be performed at once e.g. lipase or a fragment thereof, a protease or a fragment thereof and an amylase or a fragment thereof and agent, or pancreatin and agent, can be provided in one solution. The immobilization of the protein e.g. the lipase or a fragment thereof, the protease or a fragment thereof and the amylase or a fragment thereof on the solid carrier in step b) of the present method is usually carried out by adding a solution of the protein e.g. the lipase or a fragment thereof, the protease or a fragment thereof and the amylase or a fragment thereof, or pancreatin or a solution thereof, to the suspension of the solid carrier. Preferably a linker to connect the solid carrier with the protein e.g. the lipase or a fragment thereof, the protease or a fragment thereof and the amylase or a fragment thereof is added to the suspension of the solid carrier prior to adding the solution of the protein to the suspension of the solid carrier. In a preferred embodiment the immobilization of the protein e.g. the lipase or a fragment thereof, the protease or a fragment thereof and the amylase or a fragment thereof on the solid carrier is carried out by providing a suspension of the solid carrier and adding a solution of the protein e.g. the lipase or a fragment thereof, the protease or a fragment thereof and the amylase or a fragment thereof,or pancreatin or a solution thereof, wherein the suspension with the added solution of the protein e.g. the lipase or a fragment thereof, the protease or a fragment thereof and the amylase or a fragment thereof, or pancreatin or a solution thereof is incubated to allow the protein e.g. the lipase or a fragment thereof, the protease or a fragment thereof and the amylase or a fragment thereof to bind on the surface of the solid carrier.

In a more preferred embodiment the immobilization of the protein e.g. the lipase or a fragment thereof, the protease or a fragment thereof and the amylase or a fragment thereof on the solid carrier in step (e) is carried out by i) adding a linker to the solid carrier provided in step (a), preferably adding a linker to a suspension of the solid carrier provided in step a), and ii) adding the protein e.g. the lipase or a fragment thereof, the protease or a fragment thereof and the amylase or a fragment thereof provided in step (b), preferably adding a solution of the protein e.g. the lipase or a fragment thereof, the protease or a fragment thereof and the amylase or a fragment thereof provided in step (b), to the solid carrier and the linker or to the suspension comprising the solid carrier and the linker, wherein the linker connects the solid carrier with the protein e.g. the lipase or a fragment thereof, the protease or a fragment thereof and the amylase or a fragment thereof in step (e). In one embodiment, a building block of the protective layer, preferably a monomer of a building block of the protective layer, more preferably an organosilane, even more preferably a triethoxysilane, in particular APTES, is added to the solid carrier and the linker or to the suspension comprising the solid carrier and the linker, prior to adding the solution of the protein e.g. the lipase or a fragment thereof, the protease or a fragment thereof and the amylase or a fragment thereof.

In a preferred embodiment the surface of the solid carrier is at least partly modified to improve immobilization of the protein e.g. the lipase or a fragment thereof, the protease or a fragment thereof and the amylase or a fragment thereof on the solid carrier. In particular, the surface of the solid carrier is at least partly modified before the protein e.g. the lipase or a fragment thereof, the protease or a fragment thereof and the amylase or a fragment thereof is immobilized. The surface of the solid carrier can be at least partly modified by adding a molecule as anchoring point for the protein e.g. the lipase or a fragment thereof, the protease or a fragment thereof and the amylase or a fragment thereof to the surface of the solid carrier as described supra.

The suspension comprising the solid carrier is usually incubated after each addition step described above to allow a reaction between e.g. the solid carrier and the molecule as anchoring point, the solid carrier and the linker and, the solid carrier comprising the linker and the protein e.g. the lipase or a fragment thereof, the protease or a fragment thereof and the amylase or a fragment thereof, respectively, so that the the protein e.g. the lipase or a fragment thereof, the protease or a fragment thereof and the amylase or a fragment thereof connects the solid carrier, preferably the surface of the solid carrier, with the the protein e.g. the lipase or a fragment thereof, the protease or a fragment thereof and the amylase or a fragment thereof via the linker, preferably by covalent binding, thereby immobilizing the protein e.g. the lipase or a fragment thereof, the protease or a fragment thereof and the amylase or a fragment thereof on the solid carrier.

In one embodiment in step (e) the protein e.g. the lipase or a fragment thereof, the protease or a fragment thereof and the amylase or a fragment thereof is immobilized on the solid carrier by connecting the solid carrier with the protein e.g. the lipase or a fragment thereof, the protease or a fragment thereof and the amylase or a fragment thereof via a linker, preferably by connecting the solid carrier with the protein e.g. the lipase or a fragment thereof, the protease or a fragment thereof and the amylase or a fragment thereof via a linker, wherein the solid carrier is connected with the protein e.g. the lipase or a fragment thereof, the protease or a fragment thereof and the amylase or a fragment thereof by covalent binding between the linker and the solid carrier and between the linker and the protein e.g. the lipase or a fragment thereof, the protease or a fragment thereof and the amylase or a fragment thereof. Preferably i) a linker is added to solid carrier provided in step (a), and ii) the protein e.g. the lipase or a fragment thereof, the protease or a fragment thereof and the amylase or a fragment thereof provided in step (b) is added to the solid carrier and the linker, wherein the linker connects the solid carrier with the protein e.g. the lipase or a fragment thereof, the protease or a fragment thereof and the amylase or a fragment thereof in step (e). The linker used is as described supra and connects the surface of the solid carrier with the protein e.g. the lipase or a fragment thereof, the protease or a fragment thereof and the amylase or a fragment thereof by preferably covalent binding. More preferably the linker is added to the solid carrier provided in step (a), in a molar excess to the protein e.g. the lipase or a fragment thereof, the protease or a fragment thereof and the amylase or a fragment thereof provided in step (b), preferably the linker is added to the solid carrier in step (b), in a 1 fold to 1000 fold molar excess to the protein e.g. the lipase or a fragment thereof, the protease or a fragment thereof and the amylase or a fragment thereof provided in step (b), more preferably the linker is added to the solid carrier in step (b), in a 2 fold to 300 fold molar excess to the protein e.g. the lipase or a fragment thereof, the protease or a fragment thereof and the amylase or a fragment thereof provided in step (b), even more preferably the linker is added to the solid carrier in step (b), in a 4 fold to 250 fold molar excess to the protein e.g. the lipase or a fragment thereof, the protease or a fragment thereof and the amylase or a fragment thereof provided in step (b), in particular the linker is added to the solid carrier in step (b), in a nine fold molar excess to the protein e.g. the lipase or a fragment thereof, the protease or a fragment thereof and the amylase or a fragment thereof provided in step (b).

In a preferred embodiment the linker which has not connected the solid carrier with the protein e.g. the lipase or a fragment thereof, the protease or a fragment thereof and the amylase or a fragment thereof in step (e), is present during formation of a protective layer on the surface of the solid carrier in step (f). In a more preferred embodiment the linker which has not connected the solid carrier with the protein e.g. the lipase or a fragment thereof, the protease or a fragment thereof and the amylase or a fragment thereof in step (e), or a part therof, covalently binds the protective layer to the protein e.g. the lipase or a fragment thereof, the protease or a fragment thereof and the amylase or a fragment thereof in step (f). In a furthermore preferred embodiment the linker which has not connected the solid carrier with the protein e.g. the lipase or a fragment thereof, the protease or a fragment thereof and the amylase or a fragment thereof in step (e) is not removed in step (e) or step (f) or in between step (e) and (f). In a particular embodiment the linker which has not connected the solid carrier with the protein e.g. the lipase or a fragment thereof, the protease or a fragment thereof and the amylase or a fragment thereof in step (e) is not removed in step (e) or step (f) or in between step (e) and (f) and the linker which has not connected the solid carrier with the protein e.g. the lipase or a fragment thereof, the protease or a fragment thereof and the amylase or a fragment thereof in step (e), or a part thereof, covalently binds the protective layer to the protein e.g. the lipase or a fragment thereof, the protease or a fragment thereof and the amylase or a fragment thereof in step (f). The amount of the linker which has not connected the solid carrier with the protein e.g. the lipase or a fragment thereof, the protease or a fragment thereof and the amylase or a fragment thereof in step (e) after addition of the protein in ii), is usually between 30% and 70%, preferably between 40% and 60 %, more preferably around 50% of the amount of linker added to the solid carrier in step (e). In one embodiment there is no washing step between adding the linker to the solid carrier provided in step (a) in (i) and adding the protein e.g. the lipase or a fragment thereof, the protease or a fragment thereof and the amylase or a fragment thereof to the solid carrier and the linker in ii). In one embodiment there is no washing step between any of steps (a) to (f). In one embodiment there is no washing step between adding the linker to the solid carrier provided in step (e) in (i) and adding the protein e.g. the lipase or a fragment thereof, the protease or a fragment thereof and the amylase or a fragment thereof to the solid carrier and the linker in ii) and there is no washing step between any of steps (a) to (f).

In one embodiment the linker is selected from the group consisting of glutaraldehyde, disuccinimidyl tartrate, bis[sulfosuccinimidyl]suberate, ethylene glycolbis(sulfosuccinimidylsuccinate), dimethyl adipimidate, dimethyl pimelimidate, sulfosuccinimidyl (4-iodoacetyl) aminobenzoate, 1,5-difluoro-2,4-dinitrobenzene, activated sulfhydrils, sulfhydryl-reactive 2-pyridyldithiol, BSOCOES (Bis[2-(succinimidooxycarbonyloxy)ethyl]sulfone), DSP (Dithiobis[succinimidyl]propionate]), DTSSP (3,3 '-Dithiobis[sulfosuccinimidyl]propionate]), DTBP (Dimethyl 3,3 '-dithiobispropionimidate 2 HCl), DST (Disuccinimidyl tartarate), Sulfo-LC-SMPT (4-Sulfosuccinimidyl-6-methyl-a-(2-pyridyldithio)toluamido]hexanoate)), SPDP (N-Succinimidyl 3-(2-pyridyldithio)-propionate), LC-SPDP (Succinimidyl 6-(3-[2-pyridyldithio]-propionamido)hexanoate), SMPT (4-Succinimidyloxycarbonyl-methyl-a-[2-pyridyldithio]toluene), DPDPB (1,4-Di-[3'-(2'-pyridyldithio)-propionamido]butane), DTME (Dithio-bismaleimidoethane), BMDB (1,4 bismaleimidyl-2,3-dihydroxybutane) and is preferably glutaraldehyde.

In a preferred embodiment the linker is selected from the group consisting of glutaraldehyde, disuccinimidyl tartrate, bis[sulfosuccinimidyl]suberate, ethylene glycolbis(sulfosuccinimidylsuccinate), dimethyl adipimidate, dimethyl pimelimidate, sulfosuccinimidyl (4-iodoacetyl) aminobenzoate, 1,5-difluoro-2,4-dinitrobenzene, BSOCOES (Bis[2-(succinimidooxycarbonyloxy)ethyl]sulfone), DSP (Dithiobis[succinimidyl]propionate]), DTSSP (3,3 '-Dithiobis[sulfosuccinimidyl]propionate]), DTBP (Dimethyl 3,3 '-dithiobispropionimidate 2 HCl), DST (Disuccinimidyl tartarate), BMDB (1,4 bismaleimidyl-2,3-dihydroxybutane) and is preferably glutaraldehyde.

The formation of the protective layer according to step (f) of the present method is usually carried out by forming the respective protective layer with building blocks, wherein the building blocks build the protective layer in a polycondensation reaction as described supra. The immobilization of a functional constituent on the surface of the protective layer according to step (g) of the present method is usually carried out as described supra. The nanoparticles obtained in (g) are provided usually in suspension in a buffer, preferably in a histidine buffer prior to addition of the amino acid of b); the non-reducing sugar and/or the non-reducing sugar alcohol of c) and the surfactant of d). The optional lyophilization of the thus obtained suspension is usually done using a liquid nitrogen freezer and the obtained dry pellets are further blended to obtain a dry powder; which is optionally loaded into capsules preferably enterically coated capsules by conventional means.

In one embodiment the present invention provides a method of producing a nanoparticle composition comprising a) a nanoparticle comprising a solid carrier, a lipase or a fragment thereof immobilized on the surface of the solid carrier wherein the lipase or a fragment thereof is in the open conformation, a protease or a fragment thereof immobilized on the surface of the solid carrier, an amylase or a fragment thereof immobilized on the surface of the solid carrier, an agent which interacts with the lid domain of the lipase or a fragment thereof, a protective layer to protect the lipase or a fragment thereof, the protease or a fragment thereof and the amylase or a fragment thereof by embedding the lipase or a fragment thereof, the protease or a fragment thereof and the amylase or a fragment thereof, and a functional constituent immobilized on the surface of the protective layer, wherein the functional constituent immobilized on the surface of the protective layer is a polymer comprising repeat units wherein each repeat unit comprises at least one amino group and/or at least one thiol group; b) an amino acid; c) a non-reducing sugar and/or a non-reducing sugar alcohol; and d) a surfactant; the method comprising the following steps:
(a) providing a solid carrier;
(b) providing a lipase or a fragment thereof, a protease or a fragment thereof and an amylase or a fragment thereof;
(c) immobilizing the lipase or a fragment thereof, the protease or a fragment thereof and the amylase or a fragment thereof on the solid carrier;
(d) providing an agent which interacts with the lid domain of a lipase or a fragment thereof;
(e) allowing the lipase or a fragment thereof of (b) to interact with the agent of (d);
(f) forming a protective layer on the surface of the solid carrier to protect the lipase or a fragment thereof, the protease or a fragment thereof and the amylase or a fragment thereof immobilized on the solid carrier;
(g) immobilizing a functional constituent on the surface of the protective layer to obtain the nanoparticle, wherein the functional constituent immobilized on the surface of the protective layer is a polymer comprising repeat units, wherein each repeat unit comprises at least one amino group and/or at least one thiol group;
(h) providing the nanoparticles obtained in (g) in suspension and adding the amino acid of b); the non-reducing sugar and/or the non-reducing sugar alcohol of c) and the surfactant of d); and optionally;
(j) lyophilizing the supspension to obtain dry pellets and further optionally blending the dry pellets to obtain a dry powder; and optionally
(k) loading the dry powder into capsules, preferably enterically coated capsules.

In a preferred embodiment, pancreatin comprising a lipase or a fragment thereof, a protease or a fragment thereof and an amylase or a fragment thereof is provided in step b). Steps (a) to (g) can be carried out analogously to steps (a) to (g) of the method provided in the further aspect of the present invention described supra, considering that step (c) of the above method corresponds to step (e) of the method provided in the further aspect of the present invention described supra.

In one embodiment the protective layer is formed by building blocks, wherein as building blocks structural building blocks and protective building blocks are used to form the protective layer, wherein the structural building blocks are precursors of inorganic silica, capable of forming 4 covalent bonds in the layer formed and the protective building blocks are organosilanes as described above.

In one embodiment the protective layer embeds fom about 30% to about 100% of the protein e.g. the lipase or a fragment thereof, the protease or a fragment thereof and the amylase or a fragment thereof.

In one embodiment the solid carrier is selected from the group of organic particles, inorganic particles, organic-inorganic particles, self-assembled organic particles, silica particles, gold particles, magnetic particles and titanium particles and is preferably a silica particle, more preferably a silica nanoparticle (SNP).

A preferred method of the present invention is a method of producing a nanoparticle composition comprising a) a nanoparticle comprising a solid carrier, a lipase or a fragment thereof immobilized on the surface of the solid carrier wherein the lipase or a fragment thereof is in the open conformation, a protease or a fragment thereof immobilized on the surface of the solid carrier, an amylase or a fragment thereof immobilized on the surface of the solid carrier, an agent which interacts with the lid domain of the lipase or a fragment thereof, a protective layer to protect the lipase or a fragment thereof, the protease or a fragment thereof and the amylase or a fragment thereof by embedding the lipase or a fragment thereof, the protease or a fragment thereof and the amylase or a fragment thereof, and a functional constituent immobilized on the surface of the protective layer, wherein the functional constituent immobilized on the surface of the protective layer is a polymer comprising repeat units wherein each repeat unit comprises at least one amino group and/or at least one thiol group; b) an amino acid; c) a non-reducing sugar and/or a non-reducing sugar alcohol; and d) a surfactant; the method comprising the following steps:
(a) providing a solid carrier, wherein the solid carrier is provided in suspension, preferably wherein the solid carrier is provided in suspension in buffer, water and/or non-ionic surfactants, more preferably wherein the solid carrier is provided in suspension in mixtures of water and non-ionic surfactants;
(b) providing a lipase or a fragment thereof, a protease or a fragment thereof and an amylase or a fragment thereof;
(c) providing an agent which interacts with the lid domain of a lipase or a fragment thereof;
(d) allowing the lipase or a fragment thereof of (b) to interact with the agent of (c);
(e) immobilizing a protein e.g. the lipase or a fragment thereof, the protease or a fragment thereof and the amylase or a fragment thereof on the solid carrier, wherein preferably the surface of the solid carrier is at least partly modified before the protein e.g. the lipase or a fragment thereof, the protease or a fragment thereof and the amylase or a fragment thereof is immobilized on the solid carrier, wherein i) a linker is added to the suspension of the solid carrier provided in step (a) or i) a linker is added to the suspension of the solid carrier provided in step (a) after the at least partly modification of the surface of the solid carrier and ii) the protein e.g. the lipase or a fragment thereof, the protease or a fragment thereof and the amylase or a fragment thereof provided in step (b), preferably a solution of the protein e.g. the lipase or a fragment thereof, the protease or a fragment thereof and the amylase or a fragment thereof provided in step (b) is added to the the suspension of the solid carrier and the linker, wherein the linker connects the solid carrier with the protein e.g. the lipase or a fragment thereof, the protease or a fragment thereof and the amylase or a fragment thereof in step (e);
(f) forming a protective layer on the surface of the solid carrier to protect the lipase or a fragment thereof, the protease or a fragment thereof and the amylase or a fragment thereof immobilized on the solid carrier, wherein the linker which has not connected the solid carrier with the lipase or a fragment thereof, the protease or a fragment thereof and the amylase or a fragment thereof in step (e), or a part thereof, covalently binds the protective layer to the lipase or a fragment thereof, the protease or a fragment thereof and the amylase or a fragment thereof;
(g) immobilizing a functional constituent on the surface of the protective layer to obtain the nanoparticle, wherein the functional constituent immobilized on the surface of the protective layer is a polymer comprising repeat units, wherein each repeat unit comprises at least one amino group and/or at least one thiol group;
(h) providing the nanoparticles obtained in (g) in suspension and adding the amino acid of b); the non-reducing sugar and/or the non-reducing sugar alcohol of c) and the surfactant of d); and optionally;
(j) lyophilizing the supspension to obtain dry pellets and further optionally blending the dry pellets to obtain a dry powder; and optionally
(k) loading the dry powder into capsules

Alternatively steps (a) to (g) can be carried out analogously to steps (a) to (g) of the method described supra considering that step (e) of the above method corresponds to step (c) of the method described supra.

Also provided is a nanoparticle composition comprising a) a nanoparticle comprising a solid carrier, a lipase or a fragment thereof immobilized on the surface of the solid carrier wherein the lipase or a fragment thereof is in the open conformation, a protease or a fragment thereof immobilized on the surface of the solid carrier, an amylase or a fragment thereof immobilized on the surface of the solid carrier, an agent which interacts with the lid domain of the lipase or a fragment thereof, a protective layer to protect the lipase or a fragment thereof, the protease or a fragment thereof and the amylase or a fragment thereof by embedding the lipase or a fragment thereof, the protease or a fragment thereof and the amylase or a fragment thereof, and a functional constituent immobilized on the surface of the protective layer, wherein the functional constituent immobilized on the surface of the protective layer is a polymer comprising repeat units wherein each repeat unit comprises at least one amino group and/or at least one thiol group; b) an amino acid; c) a non-reducing sugar and/or a non-reducing sugar alcohol; and d) a surfactant; wherein the composition is obtainable by the methods, in particular by the preferred methods of the invention as described supra.

### Examples

### Material and Methods:

### Reagents:

- Tetraethyl orthosilicate 99%(TEOS), (3-aminopropyl)-triethoxysilane (APTES), ammonium hydroxide (ACS grade, 28-30%), ethanol (ACS grade, anhydrous), glutaraldehyde (grade I, 25% in water), polysorbate 80, pancreatin (4xUSP specifications), sodium taurocholate, fluorescein isothiocyanate (FITC), potassium phosphate monobasic, potassium phosphate dibasic, acetic acid, histidine, cysteine, trehalose, olive oil, gum arabic from acacia tree, sodium chloride, sodium hydroxide, trizma base, casein, sodium carbonate, folin reagent, trichloroacetic acid (TCA), amylase activity assay kit, triton X-100 were purchased from Sigma-Aldrich were purchased from Sigma-Aldrich.
- Benzyltriethoxysilane (B, 96%), was purchased from abcr GmbH.
- Chitosan 95/500 P was purchased from Heppe Medical Chitosan GmbH.
- Hydranal - Composite 5, Hydranal - MeOH sec and Water Standards 10.0 (for KF titration) were purchased from Honeywell.
- FEDGAS pH 6, 4.5, and 3 and FeSSIF-V2 were purchased from Biorelevant.
- Eudracap^{®} functional ready-to-fill capsules were purchased from Evonik.

### Synthesis of silica nanoparticles:

Silica nanoparticles (55 nm) have been synthetized following the original Stöber process as described in WO2015/014888 A1. Briefly, ethanol (825 mL), distilled water (107.5 mL) and ammonium hydroxide (0.13 M) were mixed and stirred at 400 rpm for 1 h. TEOS (0.28 M) was added, and the solution was stirred at 400 rpm at 20°C for 22 h. The solution was then centrifuged at 20000 g for 20 min and washed successively with ethanol and water. Particle size measurement was carried out on SEM micrographs acquired at a magnification of 150000x using the image analysis software Olympus stream motion.

### Production of non-formulated NP-3 (SNP with enzyme and protective layer and functional constituent immobilized on the surface of the protective layer) in suspension:

To SNPs (10 mg/mL, 55 nm) in phosphate buffer (20 mM, pH 8), PS80 (8 mg/L) was added APTES (3.9 mM). The reaction mixture was allowed to react for 10 min at 20°C, 400 rpm. Then, glutaraldehyde (3.9 mM) was added, and the reaction mixture was stirred for 10 min at 20°C, 400 rpm. A priming was performed by adding APTES (3.9 mM) and stirring the reaction mixture for 10 min at 20°C, 400 rpm. Sodium taurocholate (2 mM) and pancreatin (20 g/L) were sucessively added and the reaction mixture was allowed to react for 10 min at 20°C, 400 rpm. An organosilica layer was grown at the surface of the immobilized pancreatin using APTES (7.7 mM), TEOS (40.4 mM) and benzyltriethoxysilane (35 mM). The resulting suspension was allowed to react for 5 hours at 20°C, 400 rpm. The particles were centrifuged (5 min at 1000 rcf) and washed 3 times (by centrifugation during 5 min at 1000 rcf) in phosphate buffer (20 mM, pH 8), PS80 (8 mg/L) and resuspended in phosphate buffer (20 mM, pH 8), PS80 (8 mg/L). A solution of chitosan in acetic acid (0.1 M) was added to the particle suspension to achieve a final chitosan concentration of 121 µg/mL. The reaction mixture was allowed to react for 30 min at 20°C, 400 rpm.

The particles were centrifuged (5 min at 1000 rcf) and washed 3 times in phosphate buffer (0.120 M, pH 6), PS80 (8 mg/L) and resuspended in phosphate buffer (0.120 M, pH 6), PS80 (8 mg/L) to yield enzyme protected and functionalized nanoparticle NP-3 in suspension. NP-3 was cured overnight in a water bath at 20°C.

### Lyostat analysis:

The formulations were analysed using the Lyostat freeze-drying microscope, equipped with Link image and data capture software in accordance with BTL SOP LAB-QAD-302. A 2 µL sample of each formulation was pipetted between a quartz slide and glass cover slip to create a 70 µm thick 'sandwich' of sample - this ensures that the sublimation front can be observed so that the critical temperature can be established. The principal information obtained from FDM analysis is the collapse (Tc) or Eutectic (Teu) point of each formulation.

### Production of NP-3-DS nanoparticle composition:

To SNPs (30 g/L, 55 nm, 1 L) in phosphate buffer (20 mM, pH 8), PS80 (8 mg/L) was added APTES (11.8 mM). The reaction mixture was allowed to react for 10 min at 20 °C, 400 rpm to yield amino-modified SNPs. To amino-modified SNPs (30 g/L) in phosphate buffer (20 mM, pH 8), PS80 (8 mg/L) was added glutaraldehyde (11.8 mM, 25% in H₂O). The reaction mixture was allowed to react for 10 min at 20 °C, 400 rpm to yield glutaraldehyde-modified silica nanoparticles SNPs-GA. To SNPs-GA (30 g/L) in phosphate buffer (20 mM, pH 8), PS80 (8 mg/L) was added APTES (11.8 mM). The reaction mixture was allowed to react for 10 min at 20 °C, 400 rpm to yield silanol-primed silica nanoparticles SNPs-P. To SNPs-P (30 g/L) in phosphate buffer (20 mM, pH 8), PS80 (8 mg/L) was added sodium taurocholate (2 mM). The suspension was stirred for 2 minutes. Then, pancreatin (60.3 g/L) was added and the reaction mixture was allowed to react for 10 min at 20 °C, 400 rpm to yield SNPs-PCN. To SNPs-PCN (30 g/L) in phosphate buffer (20 mM, pH 8), PS80 (8 mg/L) was added APTES (23.1 mM), TEOS (121.3 mM) and benzyltriethoxysilane (105 mM). The reaction mixture was allowed to react for 5 hours at 20 °C, 400 rpm. The particles were centrifuged (5 min at 1000 rcf) and the supernatant was collected. Particles were washed 3 times (by centrifugation during 5 min at 1000 rcf) in phosphate buffer (20 mM, pH 8), PS80 (8 mg/L) and resuspended in phosphate buffer (20 mM, pH 8), PS80 (8 mg/L). SNPs-PCN-ATB were cured overnight in a water bath at 20 °C. A chitosan solution (243 µL, 1500 µg/mL) in acetic acid (0.1 M) was added to the particle suspension. The reaction mixture was allowed to react for 30 min at 20 °C, 400 rpm. The particles were centrifuged (5 min at 1000 rcf) and washed 3 times in histidine buffer (125 mM), PS80 (8 mg/L) and resuspended in histidine buffer (125 mM), PS80 (8 mg/L) to obtain a suspension of NP-3-DS. To determine particle concentration, 1 mL of the particle suspension was lyophilized. Particles suspension was further diluted with histidine buffer (125 mM), PS80 (8 mg/L) to reach a concentration of 50 g/L. Cysteine (15 mM) and trehalose (20 mM) were added to the particle suspension to yield NP-3-DS.

### Production of NP-3-DP:

NP-3-DS suspension was fast-frozen using a liquid nitrogen freezer. The frozen pellets of NP-3-DS were transferred into a freeze-dryer and lyophilized to yield dry pellets. The dry pellets were blended under argon to yield a dry powder. The dry powder was loaded into enterically coated capsules in a glove bag under argon to yield NP-3-DP.

### Pancreatic lipase activity assay:

The olive oil solution was prepared by mixing olive oil / gum ararbic / water (1 / 8.25 / 0.75). The buffer solution was prepared by dissolving Trizma base (0.6 g/L) and sodium chloride (2.34 g/L) in nanopure water. The bile salts solution was prepared by dissolving sodium taurocholate (80 g/L) in water.

The activity assay was performed by mixing the olive oil solution (13.8 mL), the buffer solution (11 mL), the bile salts solution (2.8 mL) and water (12.4 mL) in a bioreactor at 37°C. The pH was adjusted to 9.2 by adding a sodium hydroxide solution (0.1 M). NP-3 (1.4 mL, 10 mg/mL) was washed twice in water and added to the bioreactor. Lipase kinetics was monitored by measuring the amount of sodium hydroxide added to the reaction mixture to maintain the pH at 9 for 10 minutes.

### Pancreatic protease activity assay:

To the enzyme sample (50 µL, 0.2 mg/mL) in phosphate buffer (50 mM, pH 7.4) was added a casein solution (250 µL, 0.65% w/v). The reaction mixture was incubated for 30 minutes at 37 °C, 750 rpm. The sample was centrifuged for 5 minutes at 20000 rcf and the supernatant was collected. To the supernatant (200 µL) was added TCA (166.7 µL). The sample was incubated for 30 minutes at 37°C, 750 rpm. The sample was centrifuged for 5 minutes at 20000 rcf and the supernatant was collected. To the supernatant (200 µL) was added Na₂CO₃ (500 µL, 500 mM) and Folin reagent (100 µL, 0.5 M). The sample was incubated for 30 minutes at 37°C, 750 rpm. The absorbance was measured at 660 nm on 200 µL of the resulting solution.

### Pancreatic amylase activity assay:

NP-3 amylase activity was assessed using the Sigma amylase assay kit. In a 96-well plate, the enzyme sample (2 µL, 18.2 mg/mL) was mixed with the activity buffer (48 µL). The amylase substrate solution (100 µL) was added to the well and the sample kinetics was monitored at 37°C for 30 minutes in the spectrophotometer at λ = 405 nm.

### Collapse temperature determination:

The formulations were analysed using the Lyostat freeze-drying microscope, equipped with Link image and data capture software in accordance with BTL SOP LAB-QAD-302. A 2 µL sample of each formulation was pipetted between a quartz slide and glass cover slip to create a 70 µm thick 'sandwich' of sample - this ensures that the sublimation front can be observed so that the critical temperature can be established. The principal information obtained from FDM analysis is the collapse (T_{c}) or Eutectic (Tₑᵤ) point of each formulation.

### Volumetric Karl Fischer (KF) titration:

Volumetric KF titration was performed using Karl Fischer kit from Mettler Toledo.

First, water calibration was performed. The instrument automatically adjusted the titration parameters based on the results of the water standard. The residual moisture content of dry NP-3 was performed on at least 160 mg of powder.

### Scanning Electron Microscopy:

Nanoparticles are imaged using a Zeiss SUPRA^{®} 40VP scanning electron microscope.

A 1 µL drop of the nanoparticle sample is added on a freshly cleaned silicon wafer, dried under ambient conditions, and sputter-coated with a gold-platinum alloy for 15 s at 20 mA. Secondary electron micrographs are acquired using the InLens mode at a magnification of 150000X with an accelerating voltage of 10 kV.

### Laser Diffraction:

Particle size distribution was assessed using laser diffraction (dry dispersion, n=3). A standard was employed with the following parameters: particle refractive index (RI) 1.500, zero absorption, dispersant RI 1.000, and beads were utilized for dispersion.

### Ex-vivo mucus binding:

Fluorescent NP-3 nanoparticles (500 µg/mL) were applied to a mucus layer in a transwell in two forms: powder (NP-3-DP) and suspension (NP-3-DS). After a 1-hour incubation at 37°C, the mucus layer was sequentially washed with water, saline solution, and detergent (Triton X-100). Images of the transwells after each wash were captured and nanoparticles still binding to the mucus were quantified using Imaged.

### Capsules dissolution:

Eudracap capsule dissolution profiles were characterized in biologically relevant media: FEDGAS pH 6, 4.5, and 3 (simulating fed-state gastric fluids) and FeSSIF-V2 (simulating intestinal fluids). Capsules loaded with 0.15% FITC were incubated in 10 mL of each medium at 37°C. Aliquots of 250 µL were withdrawn at predefined time points (0, 0.25, 0.5, 0.75, 1, 2, 3, 4, 5, 6, and 24 hours) and analyzed for fluorescence intensity (λex/λem = 489/515 nm) to determine the release kinetics of FITC. Each experiment was conducted in triplicate.

### In vivo study:

The purpose of this study was to induce exocrine pancreatic insufficiency (EPI) in male beagle dogs by performing pancreatic duct ligation (PDL) and furthermore establish proof of concept for the drug product in this animal model.

The study was performed in male beagles (6 months old) from Marshall, France.
- Microchipping:
   Individual animal identification was ensured by a microchip. Each dog served as its own healthy control.
- High fat diet administration:
   High fat diet (HFD) was administered to the animals by offering a food diet with a 30% fat content at the dog's normal portion size. The diet was fed within a 15-minute window, whereafter the remaining food (if any) was weighed.
- Food intake:
   Food consumption (% of diet consumed at each meal) was recorded during the recovery phase to participate in monitoring the animal health. Furthermore, food consumption was recorded during study days.
- Anesthesia and antibiotics:
   For surgical procedures, the animals were initially sedated with methadone 0.3-0.4 mg/kg intramuscularly (IM)/intravenously (IV) in combination with dexmedetomidine 0.005-0.01 mg/kg IM/IV or butorphanol 0.3 mg/kg IM/IV in combination with dexmedetomidine 0.005-0.01 mg/kg IM/IV. Anesthesia was then induced with a propofol 1-4 mg/kg IV bolus followed by maintenance on inhalation anesthesia with isoflurane 2-4% after intubation. During anesthesia, amoxicillin (150 mg/mL) 15 mg/kg was administered subcutaneously (SC) as a prophylactic antibiotic.
- Analgesia:
   In relation to the surgical procedure, meloxicam 0.2 mg/kg IV/SC was given prior to the procedure. Buprenorphine 0.01-0.02 mg/kg IM/IV was given 4 hours post methadone treatment, and thereafter every 8 hours if assessed necessary.
   Meloxicam 0.1-0.2 mg/kg PO was administered 3-5 days post surgery.
- Pancreatic duct ligation:
   Pancreatic duct ligation (PDL) was performed by accessing the duodenum from a midline incision. The pancreas was localized in the duodenal mesentery and the pancreatic ducts were double ligated and incised.
- Dosing, Peroral (PO)
   The drug product (859 U) was administered by capsule administration followed by 5-10 mL tap water, 30 minutes before high fat diet consumption.
   Panzym (32790 U) was mixed with the food before animal feeding.
- Animal body weight monitoring
   The animals were weighed the day before surgery, every other day in the recovery phase (10-14 days), the day before dosing and then every 2 days.
- TLI measurements
   TLI were analyzed by an external laboratory on blood serum prior to surgery (as control) and 10-14 days post PDL surgery to confirm exocrine pancreatic insufficiency.
- Fecal sampling
   Feces were collected, pooled, kept at -20°C and shipped to Rothen Lab for analysis.

### Results:

### Example 1: Enzymatic activity recovery of NP-3-DP

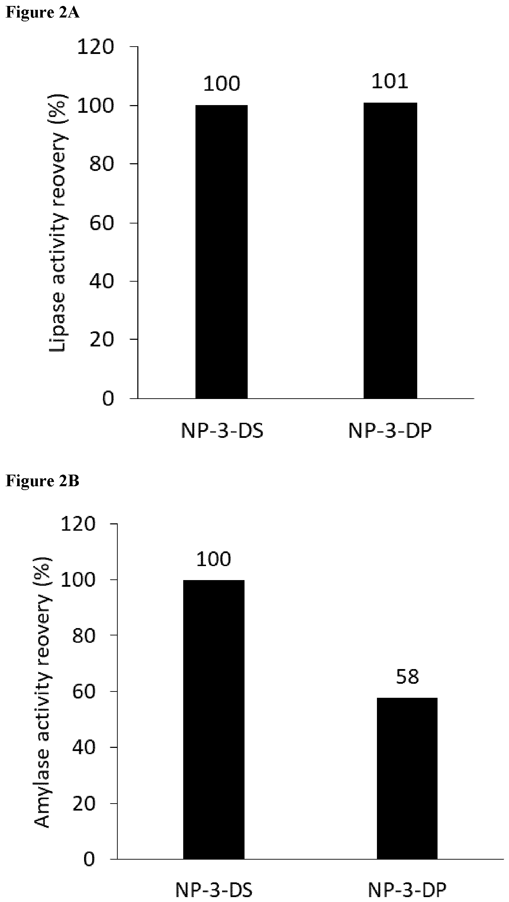

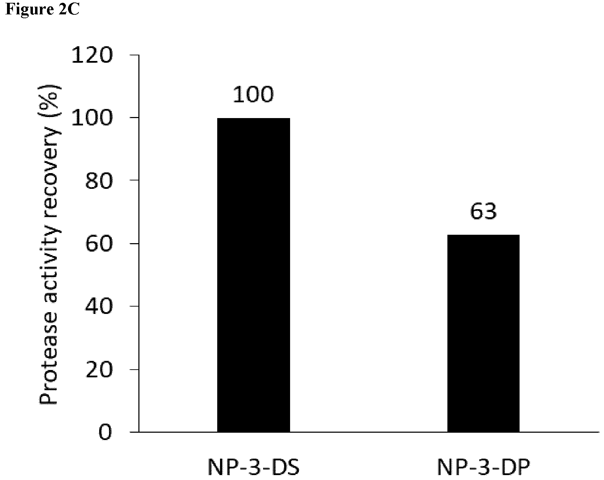

A comprehensive formulation development study was undertaken for the drug substance, involving 140 freeze-drying experiments that explored a range of freezing temperatures, buffers, and cryoprotectants. A formulation composed of 63.99% NP-3, 25% histidine, 9% trehalose, 2% cysteine, and 0.01% PS80 (w/w) emerged as the optimal composition, demonstrating excellent preservation of enzymatic activity post-freeze-drying, with 100% retention of lipase activity, 58% retention of amylase activity, and 63% retention of protease activity (Figure 2).

### Example 2: Determination of NP-3-DS collapse temperature

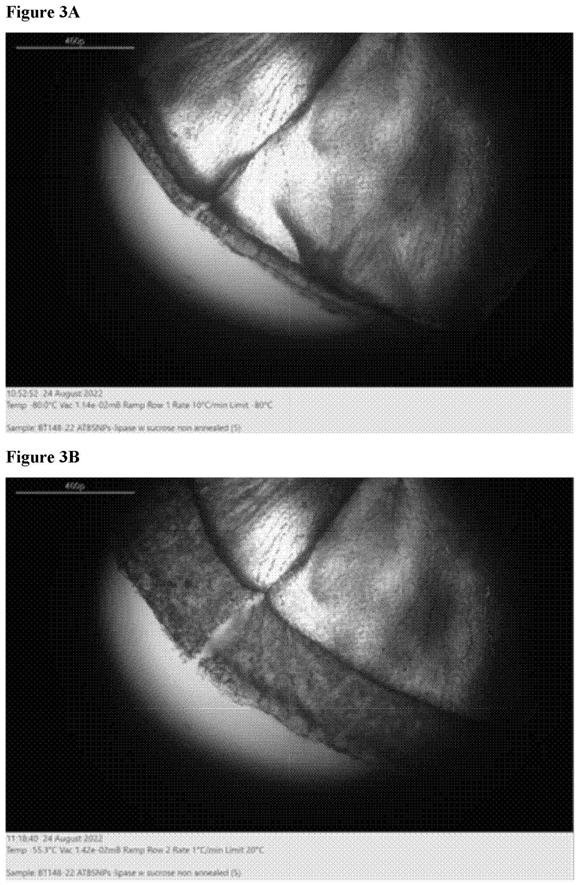

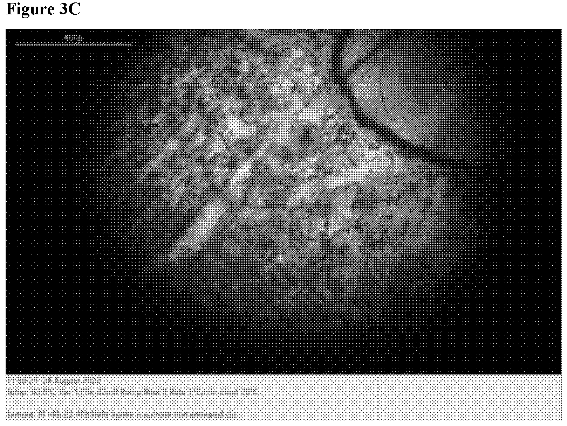

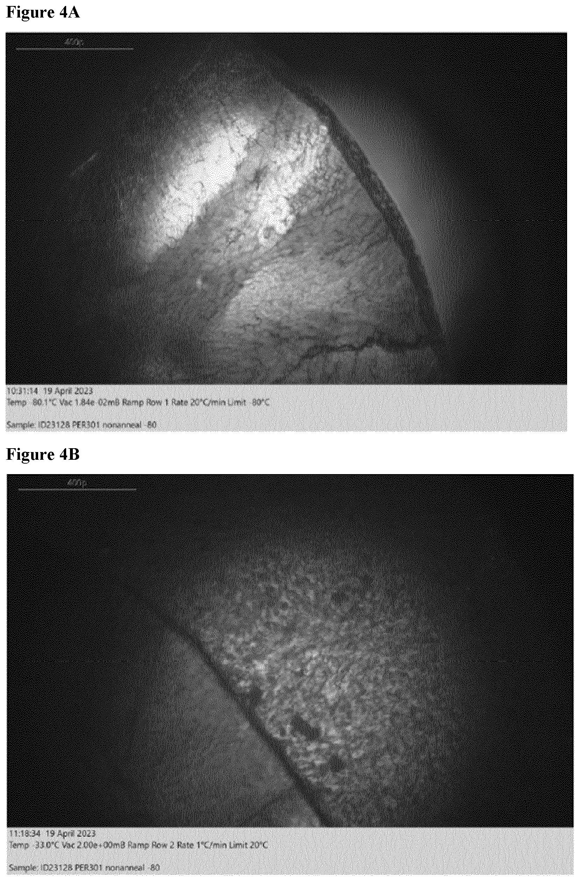

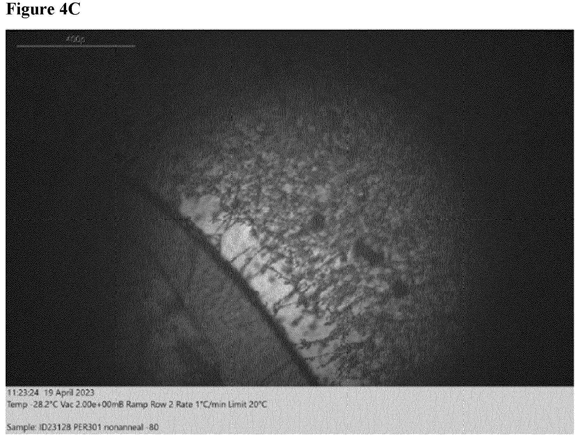

A microscopic evaluation of the freeze-drying process for NP-3-DS was conducted to assess its commercial viability. Freeze-drying microscopy analysis, using the BTL Lyostat apparatus, revealed that both non-formulated NP-3 suspension (Figure 3) and NP-3-DS (Figure 4) exhibited collapse zone behavior. This behavior is characterized by a gradual loss of structural integrity over a specific temperature range, rather than a distinct collapse point.

For non-formulated NP-3 suspension, a good structural integrity was observed at -80°C (Figure 3a). However, initial signs of collapse appeared at -55.3°C (Figure 3b), with total collapse occurring at -45.3°C (Figure 3c). In contrast, NP-3-DS maintained a good structure at -80°C (Figure 4a), showing initial signs of collapse at -33°C (Figure 4b) and total collapse at -28.2°C (Figure 4c).

Based on freeze-drying industry best practices, it is recommended to freeze formulations below the determined critical temperatures and maintain them below these temperatures until the completion of primary drying. Due to the relatively low critical temperatures of non-formulated NP-3 suspension, the development of a suitable, efficient freeze-drying cycle is considered challenging and time-consuming. The higher critical temperature observed for NP-3-DS facilitates the development of a shorter and more efficient freeze-drying cycle, making it commercially feasible.

### Example 3: Determination of NP-3-DP moisture content

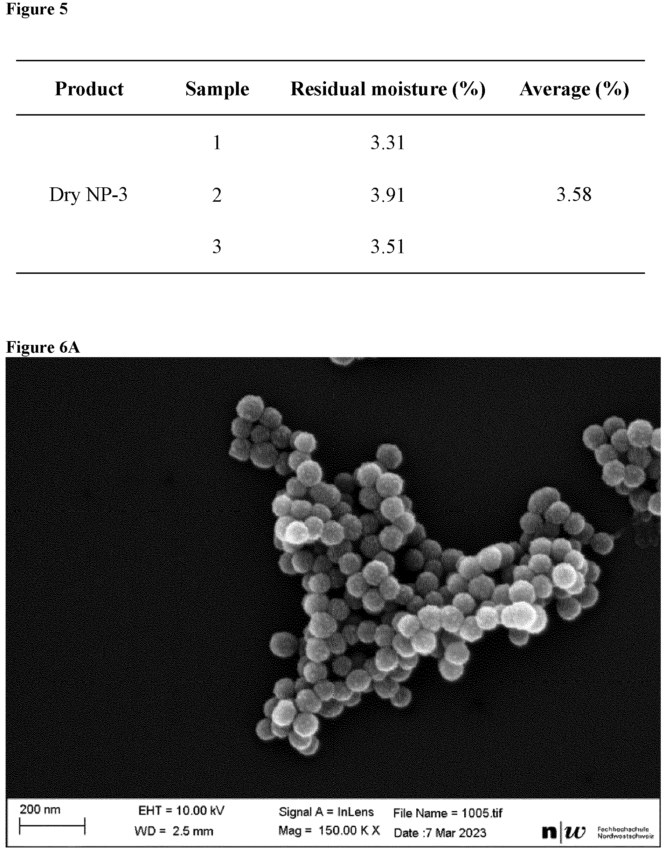

As moisture content significantly influences storage stability, Karl-Fischer titration was employed to determine the residual moisture content of NP-3-DP. Following the freeze-drying process, the residual moisture content of NP-3-DP was measured to be 3.58% (Figure 5).

### Example 4: Scanning electron microscopy of NP-3-DS and NP-3-DP

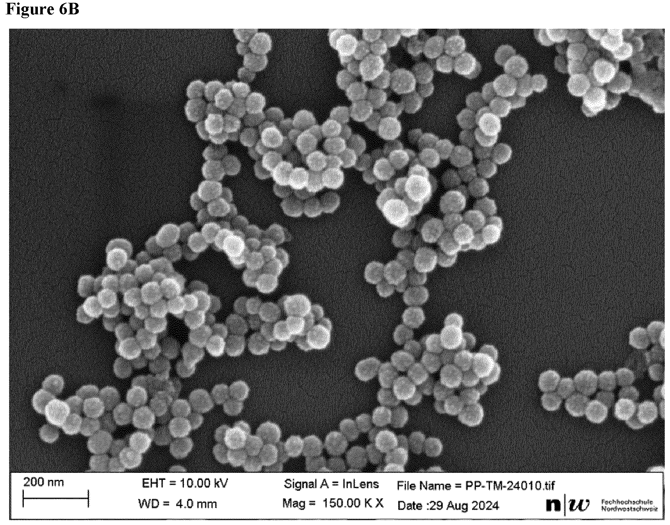

To evaluate the potential impact of the freeze-drying process on nanoparticle morphology, both NP-3-DS (Figure 6a) and NP-3-DP (Figure 6b) were examined using scanning electron microscopy. A comparative analysis revealed no discernible differences in the appearance of the nanoparticles between the two samples. These findings demonstrate that the freeze-drying process did not adversely affect the nature or shape of the nanoparticles.

### Example 5: NP-3-DP characterization

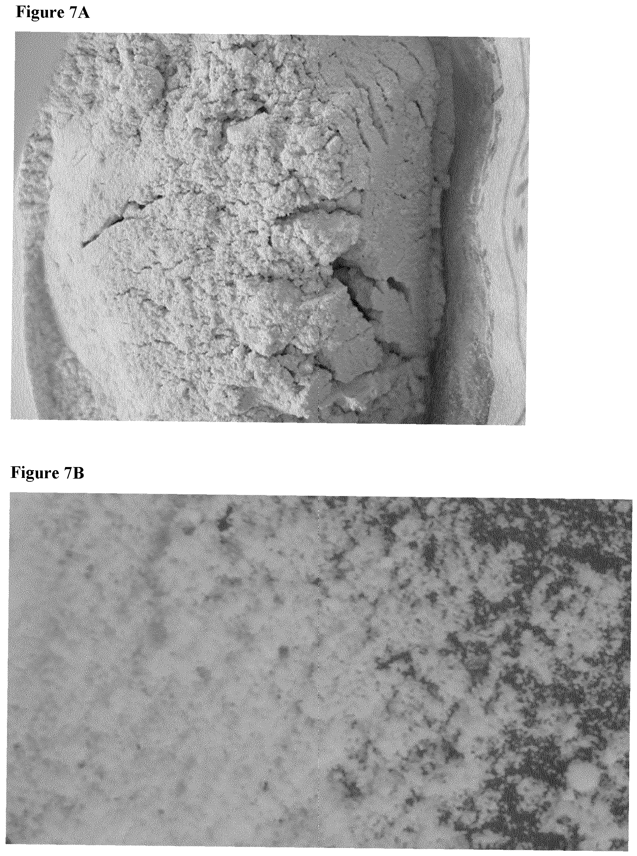

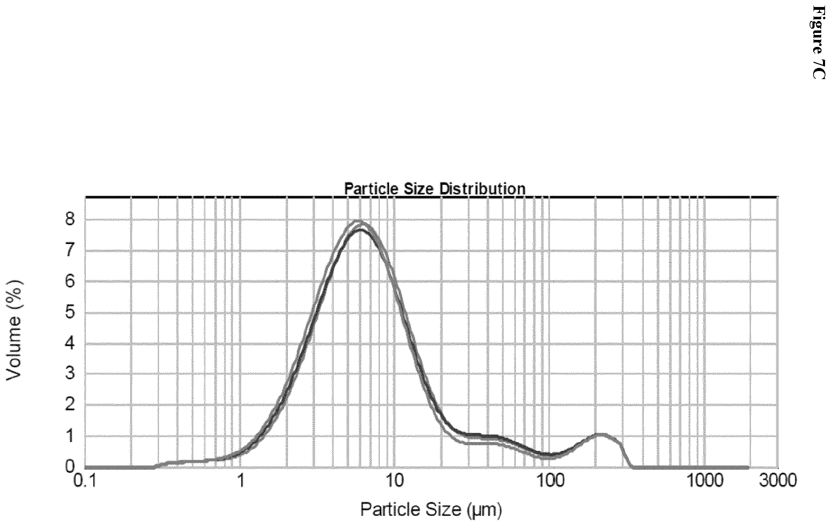

NP-3-DP appears as a fine, pinkish-beige powder (Figure 7a). Microscopic examination reveals a fine particulate nature with spherical particle morphology (Figure 7b). Laser diffraction analysis was used to characterize particle size distribution (Figure 7c). The primary particle size distribution is centered around 6 µm (D50), with a smaller population around 200 µm likely due to particle agglomeration, despite the use of dispersant beads. These findings align with microscopic observations, confirming the fine particle size of NP-3-DP, with a D10 of 2.271 µm and a D90 of 27.931 µm.

### Example 6: Impact of Freeze-Drying on Mucoadhesive Properties of NP-3-DP

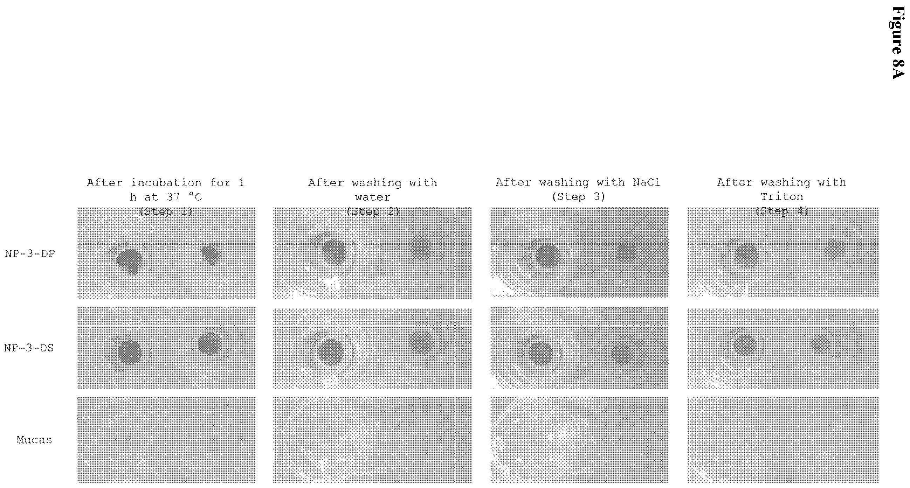

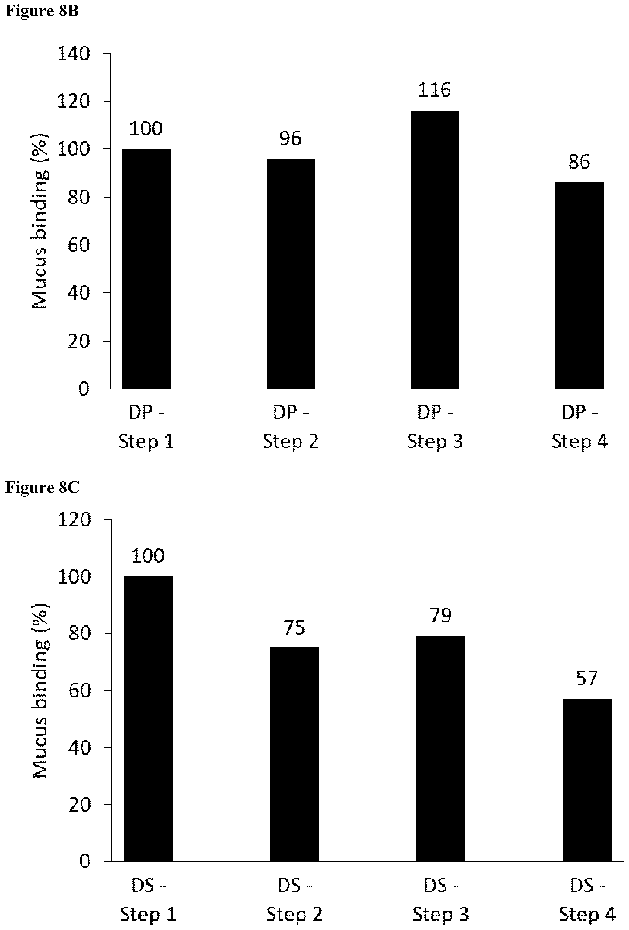

To confirm that the drying process does not compromise the mucoadhesive properties of NP-3-DS, an ex vivo study was performed using porcine intestinal mucus in a transwell system. Fluorescent NP-3 nanoparticles were applied to the mucus layer in two forms: powder (NP-3-DP) and suspension (NP-3-DS). After a 1-hour incubation period at 37°C, the mucus layer was subjected to sequential washes with water, saline solution, and detergent (Triton X-100). Images of the transwells were captured after each wash step (Figure 8a). The remaining nanoparticles bound to the mucus were quantified using ImageJ software (Figure 8b, 8c). The results indicate that NP-3-DP (Figure 8b) retains its mucoadhesive properties to a similar extent as NP-3-DS (Figure 8c), demonstrating that the drying process does not adversely affect the interaction between the nanoparticles and the mucus layer.

### Example 7: Selection of enteric capsules

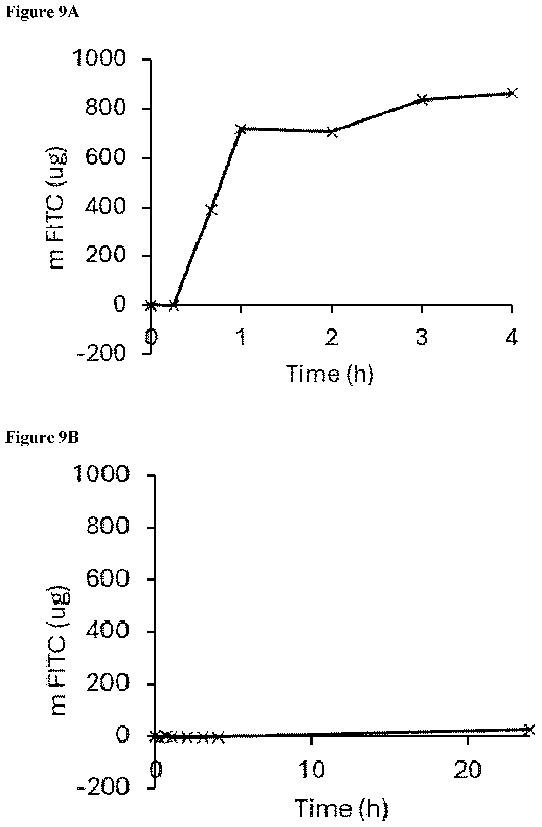

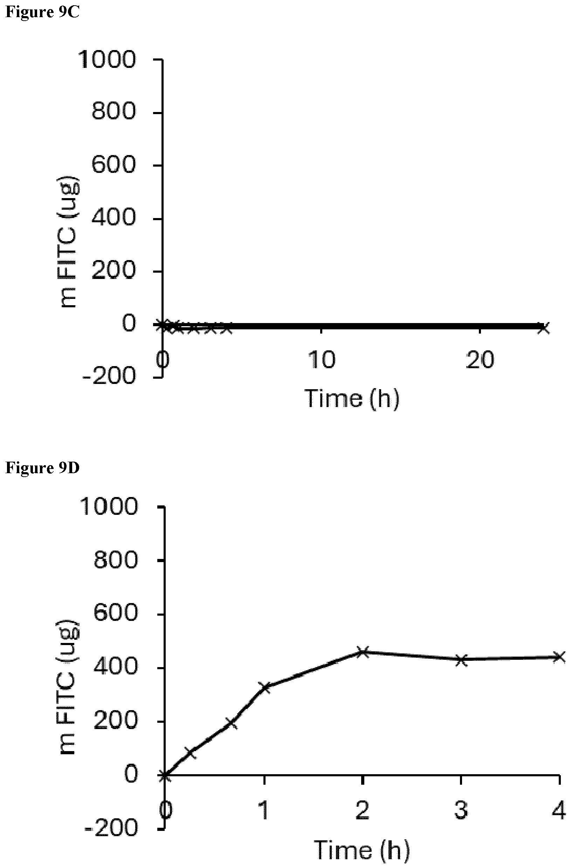

To ensure targeted drug delivery to the duodenum and protect NP-3-DP from premature release in the stomach, enteric-coated capsules were utilized. These capsules are designed to resist the acidic conditions of the stomach and dissolve at pH 6.2, the approximate pH of the duodenum. To assess the dissolution properties of Eudracap capsules, experiments were conducted in media simulating fed-state gastric fluids at pH 6 (Figure 9a), pH 4.5 (Figure 9b), and pH 3 (Figure 9c); and intestinal fluid (Figure 9d). The results indicate that NP-3-DP is not released in acidic environments but undergoes rapid release at pH 6.2, suggesting that the capsules should be administered under fasted conditions to ensure optimal drug delivery to the target site.

### Example 8: Dog weight variations

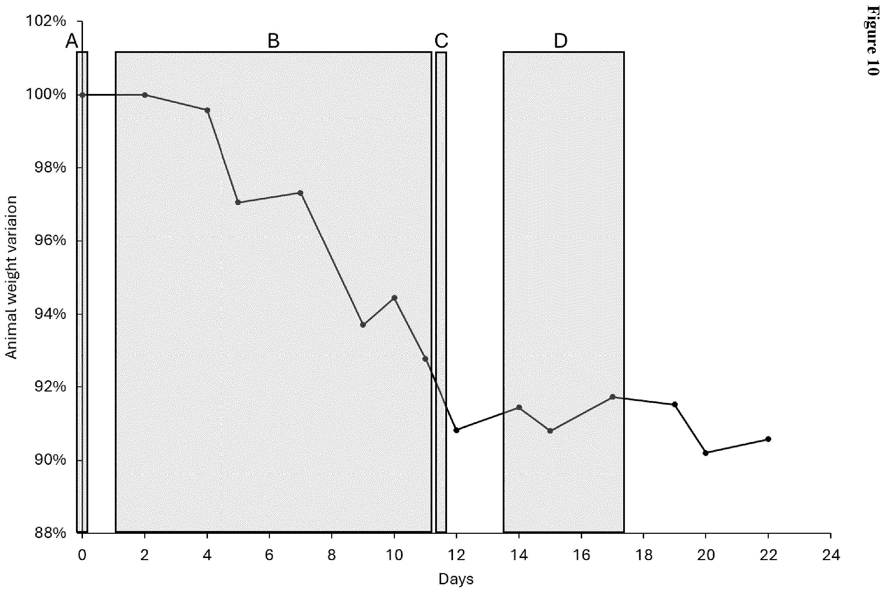

To assess the impact of NP-3-DP on digestion, body weight changes in PDL dogs were monitored. Initially, baseline weights were recorded before PDL surgery (Figure 10A). Subsequently, i.e. after surgery, the dogs were observed for 10 days without treatment, during which they experienced a 9% weight loss (Figure 10B).

On day 11, a single dose of NP-3-DP was administered (Figure 10C). This was followed by twice-daily dosing from day 14 to 16 (Figure 10D). Notably, weight loss ceased after the initial dose on day 12, demonstrating that NP-3-DP enhances digestive function.

### Example 9: Fecal fat analysis

To evaluate the digestive efficacy of NP-3-DP, fecal samples from PDL dogs were collected after a 3-day treatment period. Fecal fat content was measured and compared to samples from dogs treated with the standard of care (Panzym), untreated PDL dogs, and healthy dogs.

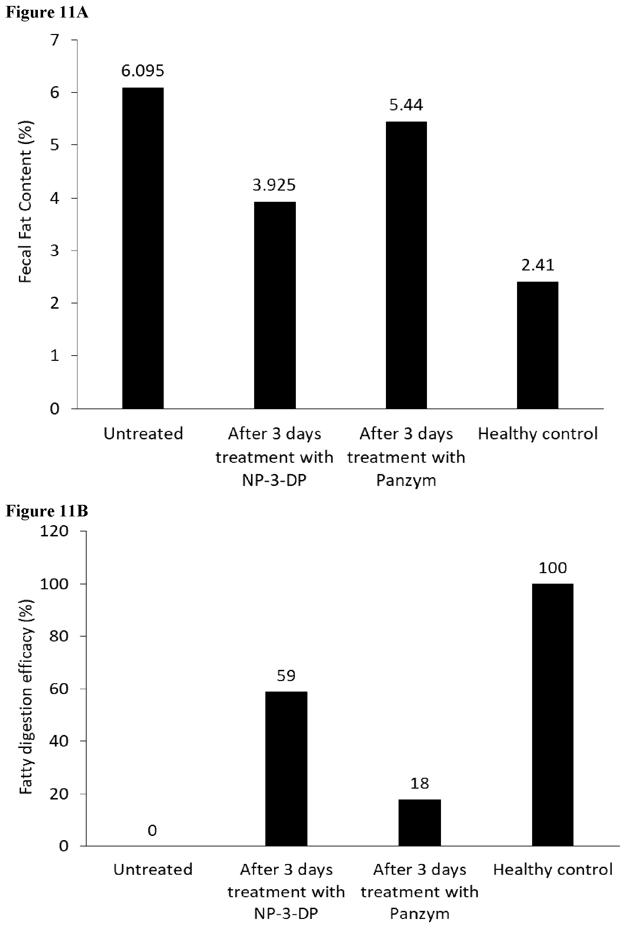

As shown in Figure 11, NP-3-DP significantly reduced fecal fat content, surpassing the performance of Panzym at a 40-fold lower dose. This rapid and substantial improvement in digestive function demonstrates a remarkable and very surprising efficacy of the product within the intestinal milieu, resulting in significantly enhanced outcomes compared to the standard of care.

## Claims

1. A nanoparticle composition comprising
a) a nanoparticle comprising a solid carrier, a lipase or a fragment thereof immobilized on the surface of the solid carrier wherein the lipase or a fragment thereof is in the open conformation, a protease or a fragment thereof immobilized on the surface of the solid carrier, an amylase or a fragment thereof immobilized on the surface of the solid carrier, an agent which interacts with the lid domain of the lipase or a fragment thereof, a protective layer to protect the lipase or a fragment thereof, the protease or a fragment thereof and the amylase or a fragment thereof by embedding the lipase or a fragment thereof, the protease or a fragment thereof and the amylase or a fragment thereof, and a functional constituent immobilized on the surface of the protective layer, wherein the functional constituent immobilized on the surface of the protective layer is a polymer comprising repeat units wherein each repeat unit comprises at least one amino group and/or at least one thiol group;
b) an amino acid;
c) a non-reducing sugar and/or a non-reducing sugar alcohol; and
d) a surfactant.

2. The composition according to claim 1, wherein the polymer comprising repeat units wherein each repeat unit comprises at least one amino group and/or at least one thiol group is a polyglucosamin selected from the group consisting of chitin, chitosan, polyglucosaminoglycans, chondroitin, heparin, keratan and dermatan or a derivative thereof.

3. The composition according to claim 1, wherein the polymer comprising repeat units wherein each repeat unit comprises at least one amino group and/or at least one thiol group is chitosan or a derivative thereof.

4. The composition of any one of claims 1-3, wherein the amino acid of b) is selected from the group consisting of alanine, arginine, cysteine, glutamic acid, glutamine, glycine, histidine, isoleucine, leucine, lysine, methionine, phenylalanine, serine, and tryptophan; the non-reducing sugar and/or non-reducing sugar alcohol of c) is selected from the group consisting of glucose, galactose, lactose, sucrose, melbiose, trehalose, mannitol, sorbitol, erythritol, glucosamine, myo-inositol, xylitol, cyclodextrins, stachyose, lactosucrose, melezidose, raffinose, alginate, carrageenan, chitosan, chondroitin sulfate, dextran, inulin, ficoll, hyaluronan, and carboxymethylcellulose; and/or the surfactant of d) is a non-ionic surfactant.

5. The composition of any one of claims 1-3, wherein the amino acid of b) is histidine and cysteine; histidine and tryptophan; histidine and methionine; glycine and cysteine; glycine and tryptophan; glycine and methionine; arginine and cysteine; arginine and tryptophan; arginine and methionine; lysine and cysteine; lysine and tryptophan; or lysine and methionine, preferably histidine and cysteine or lysine and tryptophan; the non-reducing sugar and/or non-reducing sugar alcohol of c) is selected from the group consisting of trehalose, sucrose, maltodextrin and mannitol; and the surfactant of d) is selected from the group consisting of polyvinylpyrrolidone 10K (PVP 10K), polyvinylpyrrolidone 40K (PVP 40K) and polysorbate 80 (PS80).

6. The composition of any one of claims 1-3, wherein the amino acid of b) is cysteine and histidine; the non-reducing sugar and/or non-reducing sugar alcohol of c) is trehalose; and the surfactant of d) is polysorbate 80.

7. The composition according to any of claims 1-6, wherein the nanoparticle of a) amounts from 48 percent to 81 percent by weight based on total weight of the composition, the amino acid of b) amounts from 13 percent to 32 percent by weight based on total weight of the composition, the non-reducing sugar of c) amounts from 5 percent to 12 percent by weight based on total weight of the composition and the surfactant of d) amounts from 0.005 percent to 0.02 percent by weight based on total weight of the composition, provided that the combined weight percentage of all components of the composition does not exceed 100 weight percentage of the composition.

8. The composition according to any of claims 1-6, wherein the nanoparticle of a) amounts to 63.99 percent by weight based on total weight of the composition, the amino acid of b) amounts to 27 percent by weight based on total weight of the composition, the non-reducing sugar of c) amounts to 9 percent by weight based on total weight of the composition and the surfactant of d) amounts to 0.01 percent by weight based on total weight of the composition.

9. The composition of any one of claims 1-8, wherein the composition is a solid composition, preferably a lyophilized composition.

10. The composition of any one of claims 1-9, wherein the composition is is in the form of capsules, preferably in the form of enteric-coated capsules.

11. The composition of any one of claims 1-8, wherein the composition is a liquid composition, preferably a liquid composition wherein the nanoparticles are in suspension.

12. The composition of any one of claims 1-11, for use as a medicament.

13. The composition of any one of claims 1-11, for use in a method of enzyme replacement therapy (ERT) preferably gastrointestinal enzyme replacement therapy or for use in a method for the prevention, delay of progression or treatment of exocrine pancreatic insufficiency (EPI).

14. A method of producing a nanoparticle composition, the composition comprising
a) a nanoparticle comprising a solid carrier, a lipase or a fragment thereof immobilized on the surface of the solid carrier wherein the lipase or a fragment thereof is in the open conformation, a protease or a fragment thereof immobilized on the surface of the solid carrier, an amylase or a fragment thereof immobilized on the surface of the solid carrier, an agent which interacts with the lid domain of the lipase or a fragment thereof, a protective layer to protect the lipase or a fragment thereof, the protease or a fragment thereof and the amylase or a fragment thereof by embedding the lipase or a fragment thereof, the protease or a fragment thereof and the amylase or a fragment thereof, and a functional constituent immobilized on the surface of the protective layer, wherein the functional constituent immobilized on the surface of the protective layer is a polymer comprising repeat units wherein each repeat unit comprises at least one amino group and/or at least one thiol group ; b) an amino acid;
c) a non-reducing sugar and/or a non-reducing sugar alcohol; and d) a non-ionic surfactant; the method comprising the following steps:
(a) providing a solid carrier;
(b) providing a lipase or a fragment thereof, a protease or a fragment thereof and an amylase or a fragment thereof;
(c)providing an agent which interacts with the lid domain of a lipase or a fragment thereof;
(d) allowing the lipase or a fragment thereof of (b) to interact with the agent of (c);
(e) immobilizing the lipase or a fragment thereof, the protease or a fragment thereof and the amylase or a fragment thereof on the solid carrier;
(f) forming a protective layer on the surface of the solid carrier to protect the lipase or a fragment thereof, the protease or a fragment thereof and the amylase or a fragment thereof immobilized on the solid carrier;
(g) immobilizing a functional constituent on the surface of the protective layer to obtain the nanoparticle, wherein the functional constituent immobilized on the surface of the protective layer is a polymer comprising repeat units, wherein each repeat unit comprises at least one amino group and/or at least one thiol group;
(h) providing the nanoparticles obtained in (g) in suspension and adding the amino acid of b); the non-reducing sugar and/or the non-reducing sugar alcohol of c) and the surfactant of d); and optionally;
(j) lyophilizing the supspension to obtain dry pellets and further optionally blending the dry pellets to obtain a dry powder; and optionally
(k) loading the dry powder into capsules; or
a method of producing a nanoparticle composition, the composition comprising a) a nanoparticle comprising a solid carrier, a lipase or a fragment thereof immobilized on the surface of the solid carrier wherein the lipase or a fragment thereof is in the open conformation, a protease or a fragment thereof immobilized on the surface of the solid carrier, an amylase or a fragment thereof immobilized on the surface of the solid carrier, an agent which interacts with the lid domain of the lipase or a fragment thereof, a protective layer to protect the lipase or a fragment thereof, the protease or a fragment thereof and the amylase or a fragment thereof by embedding the lipase or a fragment thereof, the protease or a fragment thereof and the amylase or a fragment thereof, and a functional constituent immobilized on the surface of the protective layer, wherein the functional constituent immobilized on the surface of the protective layer is a polymer comprising repeat units wherein each repeat unit comprises at least one amino group and/or at least one thiol group; b) an amino acid; c) a non-reducing sugar and/or a non-reducing sugar alcohol; and d) a non-ionic surfactant; the method comprising the following steps:
(a) providing a solid carrier;
(b) providing a lipase or a fragment thereof, a protease or a fragment thereof and an amylase or a fragment thereof;
(c) immobilizing the lipase or a fragment thereof, the protease or a fragment thereof and the amylase or a fragment thereof on the solid carrier;
(d) providing an agent which interacts with the lid domain of a lipase or a fragment thereof;
(e) allowing the lipase or a fragment thereof of (b) to interact with the agent of (d);
(f) forming a protective layer on the surface of the solid carrier to protect the lipase or a fragment thereof, the protease or a fragment thereof and the amylase or a fragment thereof immobilized on the solid carrier;
(g) immobilizing a functional constituent on the surface of the protective layer to obtain the nanoparticle, wherein the functional constituent immobilized on the surface of the protective layer is a polymer comprising repeat units, wherein each repeat unit comprises at least one amino group and/or at least one thiol group;
(h) providing the nanoparticles obtained in (g) in suspension and adding the amino acid of b); the non-reducing sugar and/or the non-reducing sugar alcohol of c) and the surfactant of d); and optionally;
(j) lyophilizing the supspension to obtain dry pellets and further optionally blending the dry pellets to obtain a dry powder; and optionally
(k) loading the dry powder into capsules.

15. A nanoparticle composition comprising
a) a nanoparticle comprising a solid carrier, a lipase or a fragment thereof immobilized on the surface of the solid carrier wherein the lipase or a fragment thereof is in the open conformation, a protease or a fragment thereof immobilized on the surface of the solid carrier, an amylase or a fragment thereof immobilized on the surface of the solid carrier, an agent which interacts with the lid domain of the lipase or a fragment thereof, a protective layer to protect the lipase or a fragment thereof, the protease or a fragment thereof and the amylase or a fragment thereof by embedding the lipase or a fragment thereof, the protease or a fragment thereof and the amylase or a fragment thereof, and a functional constituent immobilized on the surface of the protective layer, wherein the functional constituent immobilized on the surface of the protective layer is a polymer comprising repeat units wherein each repeat unit comprises at least one amino group and/or at least one thiol group;
b) an amino acid;
c) a non-reducing sugar and/or a non-reducing sugar alcohol; and
d) a surfactant, wherein the composition is obtainable by the method of claim 14.
